(19) **Europäisches Patentamt / European Patent Office / Office européen des brevets**

(11) **EP 4 368 140 A1**

(12) **EUROPEAN PATENT APPLICATION**
published in accordance with Art. 153(4) EPC

(43) Date of publication:
**15.05.2024  Bulletin 2024/20**

(21) Application number: **22837055.7**

(22) Date of filing: **08.07.2022**

(51) International Patent Classification (IPC):
***A61B 34/30*** (2016.01)

(52) Cooperative Patent Classification (CPC):
**A61B 34/30**

(86) International application number:
**PCT/CN2022/104671**

(87) International publication number:
**WO 2023/280310 (12.01.2023 Gazette 2023/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.07.2021  CN 202110778767**
**09.07.2021  CN 202110777345**
**09.07.2021  CN 202110778776**

(71) Applicant: **Wuhan United Imaging Healthcare Surgical**
**Technology Co., Ltd.**
**Wuhan, Hubei 430073 (CN)**

(72) Inventors:
• **ZENG, Zhixian**
**Wuhan, Hubei 430073 (CN)**

• **CHEN, Long**
**Wuhan, Hubei 430073 (CN)**
• **WANG, Xuan**
**Wuhan, Hubei 430073 (CN)**
• **LIU, Qiang**
**Wuhan, Hubei 430073 (CN)**
• **LUO, Cailian**
**Wuhan, Hubei 430073 (CN)**
• **MA, Boqi**
**Shanghai 201807 (CN)**
• **YANG, Fan**
**Wuhan, Hubei 430073 (CN)**
• **CHEN, Chaomin**
**Wuhan, Hubei 430073 (CN)**
• **XIE, Qiang**
**Shanghai 201807 (CN)**

(74) Representative: **Wang, Bo**
**Panovision IP**
**Ebersberger Straße 3**
**85570 Markt Schwaben (DE)**

(54) **SURGICAL ROBOT AND CONTROL METHOD THEREFOR**

(57)    One or more embodiments of the present disclosure relate to a surgical robot (100) and a method of controlling the surgical robot, the method comprising: obtaining pose information of an end tool; and providing first feedback information to an operator based on the pose information.

EP 4 368 140 A1

200

Pose information of an end
tool may be obtained — 210

First feedback information
may be provided to an
operator based on the pose
information — 220

In a first predetermined region,
causing the end tool to
provide second feedback
information to the operator
when the end tool moves — 230

Outputting warning
information when the end tool
fails to satisfy a
predetermined condition — 240

FIG. 2

**Description**

**CROSS REFERENCE TO RELATED APPLICATIONS**

This application claims priority to patent No. 202110778767.7, filed on July 9, 2021, claims priority to patent No. 202110777345.8, filed on July 9, 2021, and claims priority to patent No. 202110778776.6, filed on July 9, 2021, and the entire contents of each of which are incorporated herein by reference.

**TECHNICAL FIELD**

The present disclosure relates to the technical field of medical devices, and in particular, to a surgical robot and a method for controlling the surgical robot.

**BACKGROUND**

A guided robot is designed to execute a surgical procedure with an operator as a primary focus. This type of robot enhances operator involvement, effectively reducing the likelihood of an accident and improving an overall success rate of surgeries. Throughout the surgical process, the operator's proficiency in grasping pose information and control precision of an end tool of the guided robot significantly influence the success rate of surgeries. Therefore, the present disclosure provides a surgical robot and a method for controlling the surgical robot that enables the operator to timely and accurately grasp the pose information of an end tool.

**SUMMARY**

Exemplary embodiments of the present disclosure provide a method for controlling a surgical robot. The method may include obtaining pose information of an end tool and providing first feedback information to an operator based on the pose information.

Exemplary embodiments of the present disclosure provide a surgical robot. The surgical robot may include a robotic arm device configured to control an end tool to move and a positioning device configured to obtain first pose information of the end tool and second pose information of an object. The surgical robot may also include a processor configured to obtain the first pose information of the end tool and provide first feedback information to an operator based on the first pose information.

Exemplary embodiments of the present disclosure provide a method for guiding movement of an end tool. The method may include detecting a current position of an end tool, in response to determining that the current position of the end tool is not on a predetermined path in a constraint space, determining a sub-constraint space corresponding to the current position of the end tool and a distance-stiffness function corresponding to the sub-constraint space, wherein a center axis of the constraint space is the predetermined path of the end tool, and the constraint space includes at least two sub-constraint spaces nested together, and outputting a first feedback force toward the predetermined path to the end tool based on the current position and the distance-stiffness function corresponding to the current position.

Exemplary embodiments of the present disclosure provide a surgical robot. The surgical robot may include a robotic arm device configured to drive an end tool to move in response to an external force and to output a feedback force to the end tool, a positioning device configured to obtain a current position of the end tool in a constraint space, a center axis of the constraint space being a predetermined path of the end tool, the constraint space including at least two sub-constraint spaces nested together, and a processor configured to detect the current position of the end tool via the positioning device, in response to determining that the current position of the end tool is not on the predetermined path in the constraint space, determine a sub-constraint space corresponding to the current position of the end tool and a distance stiffness function corresponding to the sub-constraint space, and output a first feedback force toward the predetermined path to the end tool via the robotic arm device based on the current position and a distance-stiffness function corresponding to the current position.

Exemplary embodiments of the present disclosure provide a method for adjusting a pose of an end tool in real time. The method may include obtaining a first pose corresponding relationship between an end tool and a reference coordinate system, and a second pose corresponding relationship between the end tool and an object, obtaining a pose offset of the object in response to detecting a change of a pose of the object, wherein the pose offset of the object is a displacement of a current pose of the object relative to an initial pose of the object, and controlling the end tool to move based on the first pose corresponding relationship and the pose offset of the object so that a pose corresponding relationship between the end tool and the object is restored to the second pose corresponding relationship.

Exemplary embodiments of the present disclosure provide a surgical robot. The surgical robot may include a first pose obtaining device, provided on an object and maintaining a fixed corresponding relationship with the object, configured to obtain a pose of the object, a second pose obtaining device, provided on a robotic arm carrying an end tool or on an end tool, configured to obtain a pose of the end tool, and a controller, configured to obtain a first pose corresponding relationship between the end tool and a reference coordinate system and a second pose corresponding relationship between the end tool and the object, obtain a pose offset of the object when a change of a pose of the object is detected, and control the end tool to move based on the first pose corresponding relationship and the pose offset of the object so that a pose corresponding relationship between the end tool and the object is restored to the second pose corresponding relationship.

An embodiment of the present disclosure provides a method for automatically adjusting a pose of an acetabular cup. The method may include S1, controlling an acetabular cup to move from a current position to a predetermined cup placing position and detecting a position adjustment signal in real time, and in response to detecting the position adjustment signal, obtaining current pose information of the acetabular cup and current pose information of an acetabular socket of an object, the predetermined cup placing position being located in the acetabular socket, S2, adjusting a pose of the acetabular cup based on the current pose information of the acetabular cup and the current pose information of the acetabular socket, so as to cause a center axis of the acetabular cup to coincide with a center axis of the acetabular socket, and S3, in response to determining that the acetabular cup whose position has been adjusted is not at the predetermined cup placing position, repeating the operations S 1 and S2.

Embodiments of the present disclosure provide a surgical robot. The surgical robot may include a motion module, comprising a robotic arm configured to drive an acetabular cup to move, a pose obtaining module, comprising an acetabular cup pose unit and an acetabular socket pose unit, configured to obtain current pose information of an acetabular cup via the acetabular cup pose unit in real-time and current pose of an acetabular socket of an object via the acetabular socket pose unit in real-time, and a controller, configured to adjust a pose of the acetabular cup based on the current pose information of the acetabular cup and the current pose information of the acetabular socket so that a center axis of the acetabular cup coincides with a center axis of the acetabular socket.

## BRIEF DESCRIPTION OF THE DRAWINGS

The present disclosure is further illustrated in terms of exemplary embodiments. These exemplary embodiments are described in detail with reference to the drawings. These embodiments are not limiting, and in these embodiments, the same numbering denotes the same structure, wherein:

FIG. 1 is a schematic diagram illustrating exemplary modules of a surgical robot according to some embodiments of the present disclosure;

FIG. 2 is a flowchart illustrating an exemplary method for controlling the surgical robot according to some embodiments of the present disclosure;

FIG. 3 is a schematic diagram illustrating an exemplary first predetermined region according to some embodiments of the present disclosure;

FIG. 4 is a schematic diagram illustrating an exemplary second predetermined region according to some embodiments of the present disclosure;

FIG. 5 is a flowchart illustrating an exemplary process for applying a first feedback force according to some embodiments of the present disclosure;

FIG. 6 is a schematic diagram illustrating an exemplary image of a distance-stiffness function according to some embodiments of the present disclosure;

FIG. 7 is a flowchart illustrating an exemplary process for applying a first feedback torque according to some embodiments of the present disclosure;

FIG. 8 is a schematic diagram illustrating an exemplary image of an angle-stiffness function according to some embodiments of the present disclosure;

FIG. 9 is a flowchart illustrating an exemplary process for applying a second feedback force according to some embodiments of the present disclosure;

FIG. 10 is a schematic diagram illustrating an exemplary image of a damping function according to some embodiments of the present disclosure;

FIG. 11 is a flowchart illustrating an exemplary process for applying a second feedback torque according to some embodiments of the present disclosure;

FIG. 12 is a flowchart illustrating an exemplary process for actively adjusting a position of an end tool according to some embodiments of the present disclosure;

FIG. 13 is a flowchart illustrating an exemplary process for determining a displacement of an object according to some embodiments of the present disclosure;

FIG. 14 is a flowchart illustrating an exemplary process for determining a target displacement of the end tool according to some embodiments of the present disclosure;

FIG. 15 is a flowchart illustrating an exemplary process for calibrating an acetabular cup according to some embodiments of the present disclosure;

FIG. 16 to FIG. 19 are schematic diagrams illustrating different positions of the acetabular cup according to some embodiments of the present disclosure;

FIG. 20 is a schematic diagram illustrating an exemplary surgical robot during a joint grinding and filing phase according to some embodiments of the present disclosure;

FIG. 21 is a flowchart illustrating an exemplary process for guiding movement of the end tool according to some embodiments of the present disclosure;

FIG. 22 is a flowchart illustrating an exemplary process for adjusting a pose of the end tool in real-time according to some embodiments of the present disclosure;

FIG. 23 is a schematic diagram illustrating other exemplary modules of the surgical robot shown according to some embodiments of the present disclosure;

FIG. 24 is a flowchart illustrating an exemplary method for automatically adjusting a pose of the acetabular cup according to some embodiments of the present disclosure; and

FIG. 25 is a schematic diagram illustrating other exemplary modules of the surgical robot according to some embodiments of the present disclosure.

## DETAILED DESCRIPTION

In order to more clearly illustrate the technical solutions of the embodiments of the present disclosure, the accompanying drawings required to be used in the description of the embodiments are briefly described below. Obviously, the accompanying drawings in the following description are only some examples or embodiments of the present disclosure, and it is possible for a person of ordinary skill in the art to apply the present disclosure to other similar scenarios based on the accompanying drawings without creative labor. Unless obviously obtained from the context or the context illustrates otherwise, the same numeral in the drawings refers to the same structure or operation.

It should be understood that the terms "system", "device" as used herein, "unit," and/or "module" as used herein is a method of distinguishing between different components, elements, parts, sections, or assemblies at different levels. However, the words may be replaced by other expressions if other words accomplish the same purpose.

As used in the disclosure and the appended claims, the singular forms "a," "an," and "the" include plural referents unless the content clearly dictates otherwise. In general, the terms "including" and "comprising" suggest only the inclusion of clearly identified steps and elements, which do not constitute an exclusive list, and the method or device may also include other steps or elements.

Flowcharts are used in the present disclosure to illustrate operations performed by a system according to embodiments of the present disclosure. It should be appreciated that the preceding or following operations are not necessarily performed in an exact sequence. Instead, operations can be processed in reverse order or simultaneously. Also, it is possible to add other operations to these processes or remove one or more operations from these processes.

Embodiments of the present disclosure provide a surgical robot mainly used for performing a medical surgery such as an orthopedic surgery. In some embodiments, the surgical robot mainly includes a robotic arm device, a positioning device, and a processor, wherein the robotic arm device may be configured to control movement of an end tool.

In some embodiments, the positioning device may be configured to obtain pose information of the end tool. In some embodiments, the processor may be configured to provide first feedback information to an operator based on the pose information of the end tool. The operator may be able to learn a current position of the end tool through the first feedback information, thereby enhancing the control precision of the end tool and improving a success rate of the surgery.

In some embodiments, the positioning device may also obtain pose information of an object of a surgical operation. In some embodiments, the processor may control the end tool to proactively follow movement of the object based on the pose information of the end tool and the pose information of the object. In some embodiments, the processor may proactively adjust a pose relationship of the end tool with respect to the object based on the pose information of the end tool and the pose information of the object to satisfy a requirement of the surgery.

Embodiments of the present disclosure also provide a method for controlling a surgical robot, which may be used for controlling the surgical robot described above.

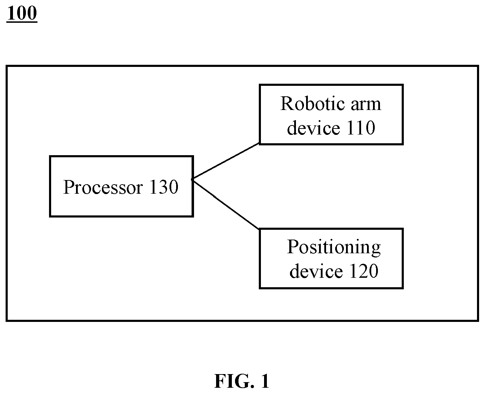

Referring to FIG. 1, FIG. 1 is a schematic diagram illustrating exemplary modules of a surgical robot according to some embodiments of the present disclosure. In some embodiments, a surgical robot 100 primarily includes a robotic arm device 110, a positioning device 120, and a processor 130.

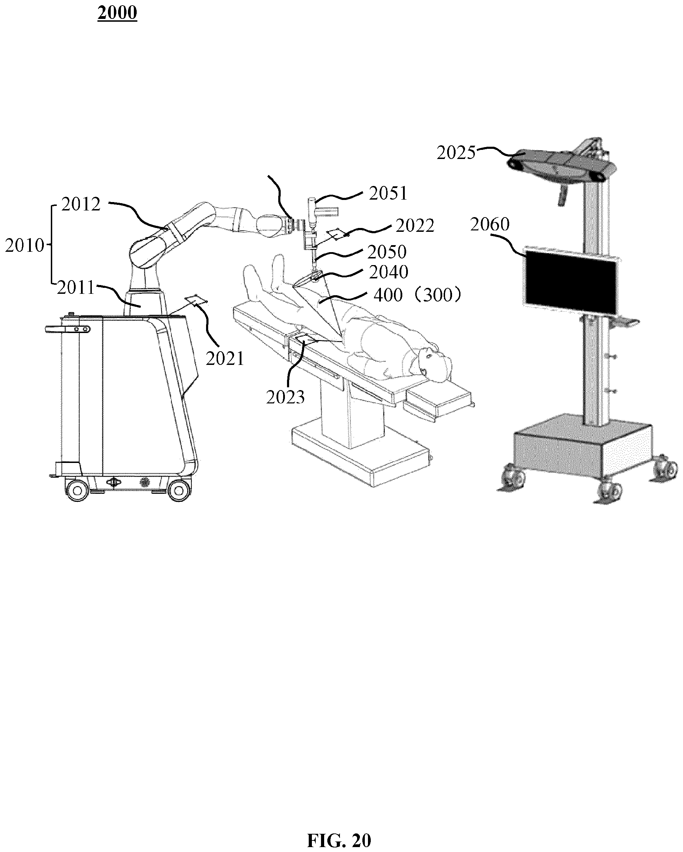

In some embodiments, the robotic arm device 110 may be configured to drive an end tool to move. In some embodiments, the robotic arm device 110 may also output a given feedback force to the end tool. More information about the robotic arm device 110 may be found with reference to FIG. 20 and related descriptions, which will not be repeated herein.

In some embodiments, the positioning device 120 (also referred to as a navigation device) may be configured to obtain pose information of a predetermined object. In some embodiments, the predetermined object may include, for example, the end tool, an object receiving a surgical operation, or other markers. In some embodiments, the positioning device 120 may be configured to obtain first pose information of the end tool and second pose information of the object. The positioning device 120 may include but is not limited to, an optical principle positioning device, an acoustic principle positioning device, an electromagnetic positioning device, a GPS positioning device, a gyroscope, an accelerometer, or the like. More information about the positioning device 120 may be found with reference to FIG. 20 and its related descriptions, and will not be repeated herein.

The processor 130 may be in data interoperability with the positioning device 120 and the robotic arm device 110, respectively. In some embodiments, the processor 130 may obtain the pose information of the end tool via the positioning device 120 and control the robotic arm device 110 to provide the first feedback information to an operator based on the pose information. In some embodiments, the processor 130 may control movement of the robotic arm device 110 thereby controlling a pose of the robotic arm device 110 and the end tool. In some embodiments, the processor 130 may include a central processing unit (CPU), a digital signal processor (DSP), a system-on-chip (SoC), a microprocessor (MCU), etc., or any combination thereof. In some embodiments, the processor 130 may be local or remote. For example, the processor 130 may access information and/or data stored in the robotic arm device 110 or other components of the surgical robot 100 (e.g., the positioning device 120) over a network. Furthermore, for example, the processor 130 may be directly coupled to the robotic arm device 110 to access information and/or data stored therein. In some embodiments, the processor 130 may be implemented on a cloud platform. By way of example only, the cloud platform may include a private cloud, a public cloud, a hybrid cloud, a community cloud, a distributed cloud, a trans-cloud, a multi-cloud, etc., or any combination thereof.

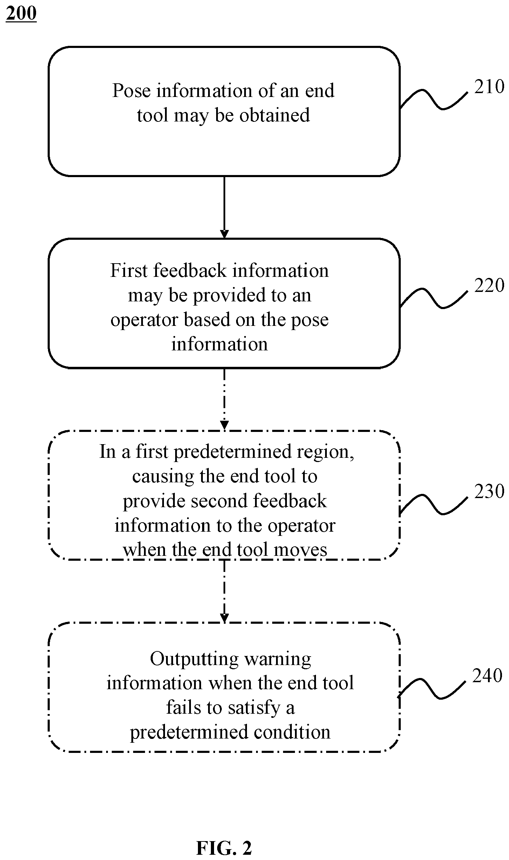

Referring to FIG. 2, FIG. 2 is a flowchart illustrating an exemplary process for controlling a surgical robot according to some embodiments of the present disclosure. As shown in FIG. 2, a process 200 includes following operations. In some embodiments, the process 200 may be performed by the surgical robot 100. Operations of process 200 presented below is illustrative. In some embodiments, the process may be accomplished utilizing one or more additional operations not described and/or one or more operations not discussed.

In 210, pose information of an end tool may be obtained. In some embodiments, operation 210 may be performed by the processor 130.

In some embodiments, the end tool may refer to an operative instrument or a treatment material, etc., disposed at an end of the robotic arm device 110 for a surgery. Exemplarily, in an orthopedic surgery, the end tool may be a file used to clean an affected area or a prosthesis used to replace a lesion. In some embodiments, the surgical robot 100 may be used in a hip surgery, and the end tool may be an acetabular file or acetabular cup.

In some embodiments, the pose information may include information such as a position and an attitude. In some embodiments, the pose information of the end tool may include a current position of the end tool. In some embodiments, the current position of the end tool may be a position of the end tool in a first predetermined region. In some embodiments, the current position of the end tool may be a distance between a feature point of the end tool and the center axis of the first predetermined region. In some embodiments, when the end tool is in the first predetermined region, the pose information of the end tool may include a current angle of the end tool. In some embodiments, the current angle of the end tool may be an angle of the end tool in the first predetermined region. In some embodiments, the current angle of the end tool may be an angle between the center axis of the end tool and the center axis of the first predetermined region. In some embodiments, the current position and/or the current angle of the end tool may also be a position or an angle in other predetermined regions, and embodiments of the present disclosure are not limiting thereon.

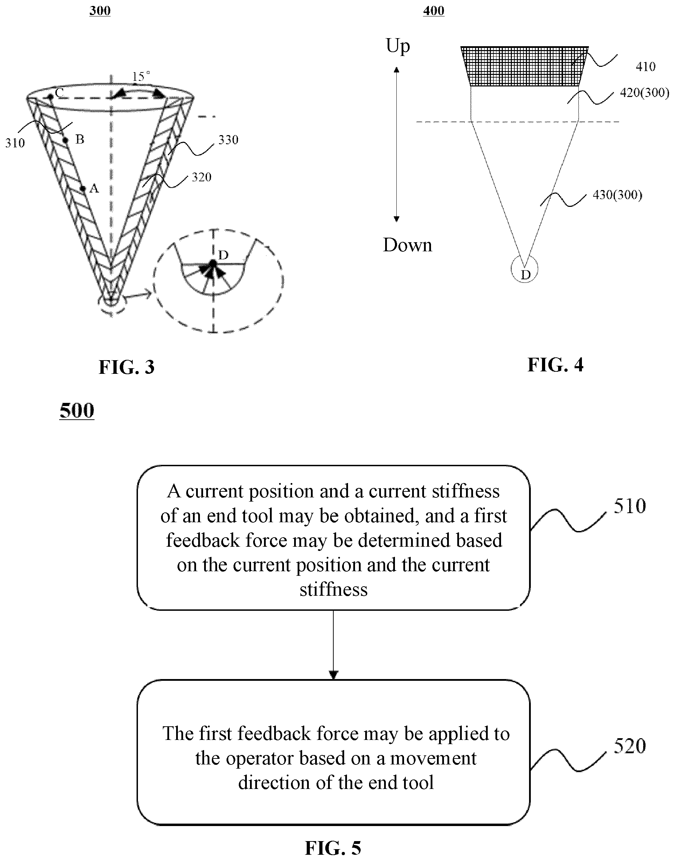

FIG. 3 is a schematic diagram illustrating an exemplary first predetermined region according to some embodiments of the present disclosure. Referring to FIG. 3, in some embodiments, an operation region of an end tool may include a first predetermined region 300, and the end tool may perform a surgical operation in the first predetermined region 300. In some embodiments, the first predetermined region 300 may be a range of regions defined by an operable space around the target position of an object receiving the surgical operation. In some embodiments, when an object who receives the surgical operation (also referred to as a surgical object or an object) moves, the first predetermined region 300 corresponding to the end tool may follow the movement of the object of the surgical operation to move. In some embodiments, the positioning device 120 may determine a pose of the end tool in the first predetermined region 300, and the processor 130 may provide first feedback information to an operator based on current pose information of the end tool to keep the end tool in the first predetermined region 300, and enable the operator to determine whether the pose of the end tool in the first predetermined region 300 satisfies a surgical requirement based on the first feedback information.

In some embodiments, the first predetermined region 300 may include a tapered region (as shown in FIG. 3). In other embodiments, the first predetermined region 300 may include a bowl-shaped region or a columnar region. In other embodiments, the first predetermined region 300 may include the tapered region and the columnar region (as shown in FIG. 4), and a central columnar region 420 in FIG. 4 may correspond to a portion of skin of the surgical subject. It should be noted that an actual range of the first predetermined region 300 may be pre-set by the operator according to a specific situation and/or a personal habit.

In some embodiments, the center axis of the first predetermined region 300 may be a predetermined path of the end tool, i.e., a movement path of the end tool desired by the operator. In some embodiments, an apex D in the first predetermined region 300 (such as the apex D in a tapered region of FIG. 3 and FIG. 4) is a target position of the end tool on the object, i.e., an end (or lower end) of the predetermined path is a surgical position of the end tool.

In some embodiments, when the end tool is located in the first predetermined region 300, the operator may manually drag the end tool to perform a surgical operation. In some embodiments, the movement range of the end tool is limited in the first predetermined region 300 when an external force applied by the operator to the end tool does not exceed a predetermined value, i.e., the movement range of the end tool is limited in the first predetermined region 300 when the external force applied by the operator to the end tool does not exceed the predetermined value, and the operator may not move the end tool beyond the first predetermined region 300. In some embodiments, the predetermined value of the external force that restricts the end tool to the first predetermined region 300 may be set by a user, for example, the user may set the predetermined value to 80 Newtons. In some embodiments, the predetermined value of the external force that restricts the end tool to the first predetermined region 300 may range from 40 Newtons to 80 Newtons. In some embodiments, the predetermined value of the external force that restricts the end tool to the first predetermined region 300 may range from 20 Newtons to 100 Newtons. In some embodiments, the predetermined value of the external force that restricts the end tool to the first predetermined region 300 may range from 60 Newtons to 80 Newtons.

In some embodiments, since the first predetermined region 300 is an operation region of the end tool, a range of a cone angle of a tapered region of the first predetermined region 300 is related to a size of an opening of the operation region, and the range of the cone angle represents a range of an allowable angle between the end tool and the predetermined path. Therefore, the operator may set the range of the cone angle of the tapered region on a case-by-case basis. In some embodiments, the range of the cone angle of the tapered region may range from 20 degrees to 40 degrees. In some embodiments, the range of the cone angle of the tapered region may range from 25 degrees to 35 degrees. In some embodiments, the cone angle of the tapered region may be 30 degrees, and the angle of a semi-cone angle of the tapered region is 15 degrees as shown in FIG. 3.

In some embodiments, the height of the tapered region of the first predetermined region 300 represents a depth of the operation region, i.e., a length distance from an opening of a surface layer of the skin of the object to the operation region (the target pose), and thus in practice, the operator may set the height range of the tapered region according to a specific situation. In some embodiments, the height range of the tapered region may be 100 millimeters to 300 millimeters. In some embodiments, the height range of the tapered region may be 150 millimeters to 250 millimeters. In some embodiments, the height of the tapered region may be 200 millimeters.

FIG. 4 is a schematic diagram illustrating an exemplary second predetermined region according to some embodiments of the present disclosure. In some embodiments, referring to FIG. 4, an operation region of an end tool may include a second predetermined region 400 where the end tool may follow movement of an object in the second predetermined region 400. In some embodiments, when the end tool is disposed in the second predetermined region 400, and when an object receiving a surgical operation moves, the robotic arm device 110 may actively control the end tool to generate a corresponding movement based on movement information of the object receiving the surgical operation. That is, when the object receiving the surgical operation moves, at this time, the robotic arm device 110 may control the end tool to actively move based on corresponding information (e.g., the movement information of the object, force information of the end tool, etc.) to ensure that a relative position between the end tool and a surgical object remains unchanged or substantially unchanged, henceforth facilitating a subsequent operation of a surgery.

In some embodiments, a range of the second predetermined region 400 may be predetermined (e.g., by an operator or a manufacturer). In some embodiments, the second predetermined region 400 may be a region within a certain range above the first predetermined region 300. In some embodiments, the second predetermined region 400 may be a region within a certain overlap range with the first predetermined region 300. In some embodiments, referring to FIG. 4, the second predetermined region 400 may be a region that contains the first predetermined region 300. It should be noted that "up" and "down" as used in the present disclosure refer relative directions, referring to FIG. 4, a side that is near a target position on the object along a direction of a center axis of the first predetermined region 300 is "down", and a side that is far away from the target position on the object along the direction of the center axis of the first predetermined region 300 is "up". In some embodiments, the shape of the second predetermined region 400 may be any shape.

In some embodiments, the second predetermined region 400 may include the first predetermined region 300. In some embodiments, as shown in FIG. 4, a top shaded region 410, a middle columnar region 420, and a lower tapered region 430 in FIG. 4 may all belong to the second predetermined region 400, and when the end tool is located in any one of the above three regions, the robotic arm device 110 may actively control the end tool to move.

In some embodiments, the second predetermined region 400 may not include the first predetermined region 300. In some embodiments, the top shaded region 410 in FIG. 4 may belong to the first predetermined region 400, and the middle columnar region 420 and the lower tapered region 430 (i.e., the first predetermined region 300) may not belong to the second predetermined region 400, and when the end tool is located in the top shaded region 410, the robotic arm device 110 may actively control the end tool to follow the movement of the object; when the end tool is located in the central columnar region 420 or the lower tapered region 430, the end tool may be controlled by the operator to follow the movement of the object.

In some embodiments, pose information of the end tool may include distance information and angle information of the end tool. The distance information of the end tool may include a target distance between the current position of the end tool and a predetermined path; and the angle information of the end tool may include a current angle between the axis of the end tool and the predetermined path (e.g., a center axis of a tapered region of the first predetermined region 300).

In some embodiments, the pose information of the end tool may be determined by the positioning device 120, and the processor 130 may obtain the pose information of the end tool via the positioning device 120.

In some embodiments, the end tool may be provided with a feature point for pose tracking , and pose information of the feature point may represent the pose information of the end tool.

In 220, first feedback information may be provided to the operator based on the pose information. In some embodiments, operation 220 may be performed by the processor 130.

In some embodiments, the operator may refer to an operator of a surgical robot, such as a physician, etc. In some embodiments, the first feedback information is determined based on the pose information of the end tool, and different pose information of the end tool corresponds to different first feedback information. The operator may determine corresponding pose information of the end tool based on the received first feedback information.

In some embodiments, the first feedback information may include a first feedback force. Operation 220 may include applying the first feedback force to the operator based on the pose information. The first feedback force is a force that is fed back to the operator, and a magnitude and direction of the first feedback force are related to the pose information and a movement direction of the end tool. In some embodiments, the first feedback force may vary non-linearly based on the pose information of the end tool to enhance the warning effect. In some embodiments, the smaller a distance between the end tool and the predetermined path is, the smaller the magnitude of the first feedback force may be. In some embodiments, the magnitude of the first feedback force may be 0 if the distance between the end tool and the predetermined path is 0. In some embodiments, the greater the distance between the end tool and the predetermined path is, the greater the magnitude of the first feedback force may be. In some embodiments, when the end tool is located at an edge of the first predetermined region 300, the distance between the end tool and the predetermined path is large, and at this time, the first feedback force may suddenly increase to warn the operator. More information about the first feedback force can be found in FIG. 5 and its related descriptions, and will not be repeated here.

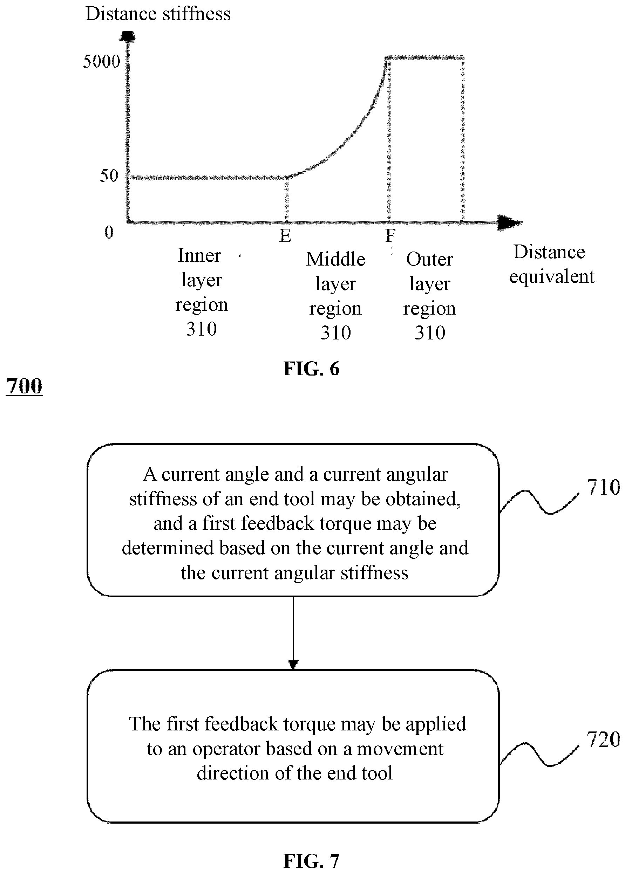

In some embodiments, the first feedback information may further include a first feedback torque. Operation 220 may further include an operation of applying the first feedback torque to the operator based on the pose information. The first feedback torque is a torque that is fed back to the operator. In some embodiments, the first feedback torque may vary non-linearly based on the pose information of the end tool in order to enhance the warning effect. More information about the first feedback torque can be found in FIG. 7 and its related description, which will not be repeated here.

In some embodiments, the process 200 may further include operation 230 including in the first predetermined region, causing the end tool to provide second feedback information to the operator when the end tool moves. In some embodiments, operation 230 may be performed by the robotic arm device 110.

In some embodiments, the processor 130 may cause the end tool to provide the second feedback information to the operator based on the movement information of the end tool when the end tool moves in the first predetermined region 300. The movement information of the end tool may include information such as a movement speed of the end tool, an angular speed of the movement, or the like.

In some embodiments, the second feedback information may be determined based on a damping coefficient. In some embodiments, the damping coefficient may be determined based on a damping function corresponding to the movement speed or angular speed of movement of the end tool. In some embodiments, one or more biometric features of the object may be used to determine the damping coefficient to simulate a realistic sensation during the surgical operation, and the second feedback information may be determined based on the one or more biometric features of the object. In some embodiments, the one or more biometric features of the object may include human body features such as fat, joint fluid, or the like.

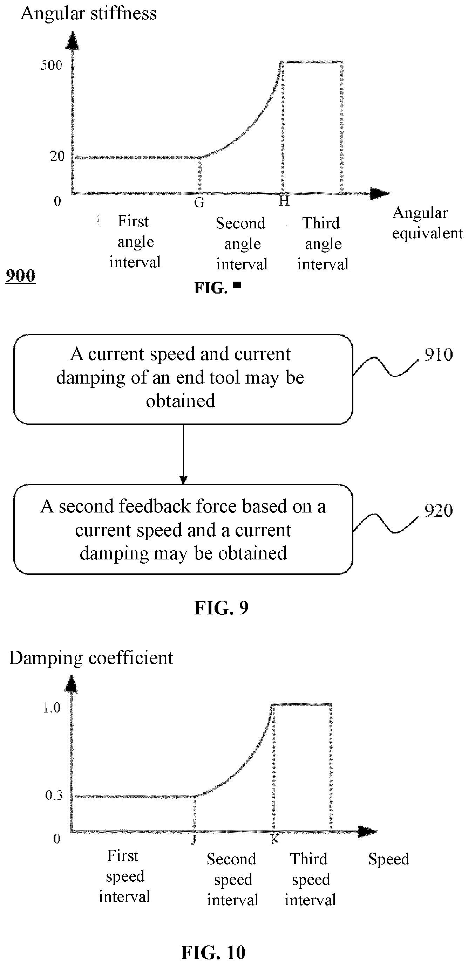

In some embodiments, the second feedback information may include a second feedback force. The second feedback force may refer to a force that is determined based on the movement speed of the end tool and is applied to the operator. More descriptions about the second feedback force can be found with reference to FIG. 9 and its related description, which will not be repeated here.

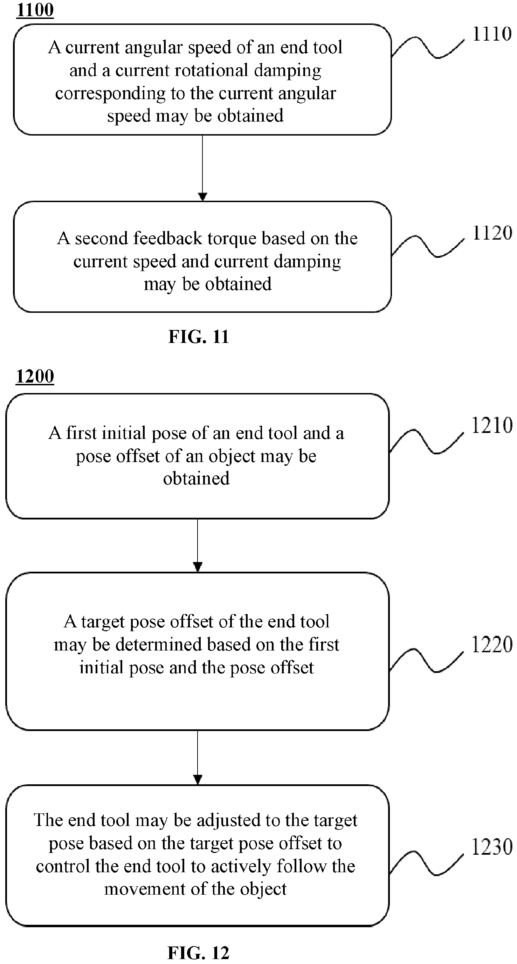

In some embodiments, the second feedback information may further include a second feedback torque. The second feedback torque may be a torque determined based on the angular speed of movement of the end tool and is applied to the operator. More descriptions about the second feedback torque can be found with reference to FIG. 11 and its related description, which will not be repeated here.

In some embodiments, process 200 may further include operation 240 including outputting warning information when the end tool fails to satisfy a predetermined condition. In some embodiments, operation 240 may be performed by the processor 110.

In some embodiments, the predetermined condition may be an interval range of a parameter or a judgment condition related to the end tool, such as a range of the magnitude of an allowable force on the end tool, whether an actual path of the end tool matches the predetermined path, or the like. In some embodiments, a count of predetermined conditions may be one or two or more. In some embodiments, the warning information is a signal used to alert the operator and draw the operator's attention. In some embodiments, the warning information may include audible warning information, visual warning information, or the like. For example, the warning information may include an alarm bell, a light of a specified color, a specified warning pattern, a light source flashing at a specified frequency, or the like. In some embodiments, the warning information may be emitted by a corresponding component, such as a speaker may emit the alarm bell, a light bulb may emit the light of a specified color or a light ray of a specified flashing frequency, or the like.

In some embodiments, operation 240 may include outputting the warning information when the end tool fails to detach from the first predetermined region according to the predetermined path. When the actual path of the end tool does not coincide with the predetermined path, the end tool detaching from the first predetermined region 300 may adversely affect a wound in an opening region of the surgical object, which in turn affects the surgery effect. Therefore, when the end tool fails to detach from the first predetermined region 300 in accordance with the predetermined path, the processor 130 may control a corresponding component to emit corresponding warning information.

In some embodiments, operation240 may further include outputting the warning information when a force or torque subjected by the end tool is greater than a predetermined threshold value. In some embodiments, the predetermined threshold value may include a maximum value of an external force subjected by the end tool or a maximum value of a torque generated by the external force subjected by the end tool. In order to avoid the end tool from detaching from the first predetermined region 300 under an action of the external force, the external force or torque applied by the operator on the end tool should be in a range of the predetermined threshold value to avoid affecting the surgical effect. Therefore, when the force or torque subjected by the end tool is greater than the predetermined threshold value, the processor 130 may control a corresponding component to emit corresponding warning information.

In some embodiments, the end tool may include a six-dimensional force sensor. The six-dimensional force sensor may be used to detect the force and/or torque subjected by the end tool, such as a contact force of the end tool with a target position on an object receiving a surgical operation, a drag force applied to the end tool by the operator, etc. In some embodiments, the six-dimensional force sensor may also perform low-pass filtering on a detected force signal to filter out a noise such as shaking of the operator's hand or shaking of the surgical object, thereby improving the accuracy of force detection.

In some embodiments, in response to detecting that the surgical object moves, the process 200 may include other operations for adjusting the end tool's pose to allow the end tool to actively follow the object to move. For more information on actively adjusting the end tool's pose, please refer to FIG. 12 and the related description, which will not be repeated here.

FIG. 5 is a flowchart illustrating an exemplary process for applying a first feedback force according to some embodiments of the present disclosure. As shown in FIG. 5, a process 500 may include following operations. In some embodiments, the process 500 may be performed by the surgical robot 100. Operations of the process 500 presented below is illustrative. In some embodiments, the process may be accomplished utilizing one or more additional operations not described and/or one or more operations not discussed.

In 510, a current position and a current stiffness of an end tool may be obtained, and a first feedback force may be determined based on the current position and the current stiffness. In some embodiments, operations 510 may be performed by the processor 130.

In some embodiments, the current position of the end tool may be a position of a feature point of the end tool in the first predetermined region 300 relative to a predetermined path. In some embodiments, based on the current position of the end tool, a current distance (also referred to as a target distance) from the feature point of the end tool to the predetermined path in the first predetermined region 300 may be determined. In some embodiments, the feature point of the end tool may be a point on the end tool used for position tracking. In some embodiments, the current stiffness of the end tool is the ability of the end tool to resist moving away from the predetermined path. The current stiffness of the end tool is related to the current position of the end tool. For example, the farther the end tool is from the predetermined path is, the greater the current stiffness of the end tool may be.

In some embodiments, a product of the target distance corresponding to the current pose and the current stiffness may be used as a magnitude of the first feedback force. In some embodiments, a direction of the first feedback force may be that the end tool is toward the predetermined path, i.e., the direction of the first feedback force may be toward the predetermined path.

In some embodiments, a corresponding relationship involves between the current stiffness of the end tool and the current position of the current pose. In some embodiments, the corresponding relationship may be in tabular form, and a distance value or a distance range may correspond to a stiffness value. In other embodiments, the corresponding relationship may also be in the form of a function, where the distance value (e.g., a current distance) may serve as an independent variable of the function and the stiffness value (e.g., the current stiffness) may serve as a dependent variable of the function, and each of the distance value may be used to determine a corresponding stiffness value based on the function.

In some embodiments, the corresponding relationship may be pre-stored in the processor 130 or may be entered temporarily. In some embodiments, the corresponding relationship be viewed or modified by an operator.

In some embodiments, the current stiffness may be determined based on a distance-stiffness function. In some embodiments, an independent variable of the distance-stiffness function may be a current distance between the current position of the end tool and the predetermined path, and the current stiffness corresponding to the current distance may be determined by the distance-stiffness function.

In other embodiments, the independent variable of the distance-stiffness function may be a distance equivalent of a feature point of the end tool at the current position in the first predetermined region 300 relative to the predetermined path. In some embodiments, the distance equivalent may be a ratio of a current distance between the feature point of the end tool at the current position and the predetermined path to a maximum distance, i.e., in a tapered region, a ratio of a distance between the feature point of the end tool and the predetermined path to a distance between a boundary of the first predetermined region and the predetermined path. After determining a distance corresponding to the current position of the end tool, the distance equivalent corresponding to the distance may be determined; based on the distance equivalent and the distance-stiffness function corresponding to the current position, the current stiffness corresponding to the current position may be determined.

FIG. 6 is a schematic diagram illustrating an exemplary image of a distance-stiffness function according to some embodiments of the present disclosure. Referring to FIG. 6, horizontal coordinates of a coordinate system shown in FIG. 6 are a current distance equivalent of an end tool, i.e., an independent variable of the distance-stiffness function is a distance equivalent. In some embodiments, the independent variable of the distance-stiffness function (e.g., the distance equivalent) may range from 0 to 1. In some embodiments, the distance-stiffness function may be a segmented function in a value interval of the independent variable of the distance-stiffness function. In other embodiments, the distance-stiffness function may be a non-segmented function in the value interval of the independent variable of the stiffness function. In some embodiments, the distance-stiffness function may include a constant function and a monotonically increasing quadratic function. In other embodiments, the distance-stiffness function may include a discrete function. In other embodiments, the distance-stiffness function may include other monotonically increasing functions, such as a cubic function, a monotonically increasing segmented function, or the like.

In some embodiments, the independent variable of the distance-stiffness function takes a distance equivalent, which may allow the end tool to be subjected to the same first feedback force at different locations on a boundary of a same region in an axial direction of the first predetermined region 300. As shown in FIG. 3, point A and point B are boundary points of an inner layer region 310, respectively, and although a distance from the point A to a center axis (a predetermined path) of the first predetermined region 300 and a distance from the point B to the center axis (the predetermined path) of the first predetermined region 300 are different, both the two points are located on the boundary of the same region, and both correspond to the same distance equivalent, and thus the first feedback force subjected by the end tool at the boundary point A is the same as the first feedback force subjected by the end tool at the boundary point B. This allows an operator to determine whether the end tool has arrived at or is adjacent to the boundary of each of regions in the first predetermined region 300 by sensing the first feedback force provided by the end tool at the current position, thereby determining a position range of the end tool.

Referring to FIG. 3, taking the inner layer region 310 of FIG. 3 as an example, a radius corresponding to a boundary point C of the inner layer region 310 of FIG. 3 is taken as a reference radius $R_{reference}$, M is set to be the distance-stiffness function, and the first feedback force subjected by the end tool at the boundary point C is $F = M \cdot R_{reference}$. In response to a determination that the current position of the end tool is located at a center of the reference radius, the first feedback force subjected by the end tool at the center $\frac{R_{reference}}{2}$ of the reference radius is

$$M \cdot \frac{R_{reference}}{2} = \frac{F}{2}$$

. Similarly, in response to a determination that the end tool is currently located on a middle point of a radius where the boundary point B is located, a maximum radius corresponding to the current position of the end tool is $R_B$, and a distance between the current position and the predetermined path is $\frac{R_B}{2}$, and the first feedback force

$$f = M \frac{R_B}{2} \frac{R_{reference}}{R_B} = M \frac{R_{reference}}{2} = \frac{F}{2}$$

subjected by the end tool at the current position is . That is, the end tool receives the same first feedback force at the current positions with the same distance equivalent.

In some embodiments, the distance-stiffness function is continuous in more than half of a value interval of the independent variable of the distance-stiffness function. In some embodiments, as shown in FIG. 6, the independent variable of the distance-stiffness function may include three continuous value intervals, and the three value intervals may correspond to the inner layer region 310, a middle layer region 320, an outer layer region 330 of the first predetermined region 300, and the distance-stiffness function may be continuous in two or three value intervals. A junction point between a first value interval (corresponding to the inner layer region 310 of the first predetermined region) and a second value interval (corresponding to the middle layer region 320 of the first predetermined region) is set to be a first junction point E. A junction point between the second value interval and a third value interval (corresponding to the outer layer region 330 of the first predetermined region) is a second junction point F. A value of the distance-stiffness function corresponding to the first junction point E of the first value interval (the inner layer region 310) and a value of the distance-stiffness function corresponding to the junction point E of the second value interval (the middle layer region 320) are the same, so that the distance-stiffness function may be continuous in the first value interval (the inner layer region 310) and the second value interval (the middle layer region 320). A value of the distance-stiffness function corresponding to the second junction point F of the second value interval (the middle layer region 320) and a value of the distance-stiffness function corresponding to the second junction point F of the third value interval (the outer layer region 330) are the same, so that the distance-stiffness function may be continuous in the second value interval (the middle layer region 320) and the third value interval (the outer layer region 330). In some embodiments, the distance-stiffness function is continuous in more than 60% of the value interval of the independent variable. In some embodiments, the distance-stiffness function is continuous in more than 80% of the value interval of the independent variable the distance-stiffness function. In some embodiments, the distance-stiffness function is continuous in more than 90% of the value interval of the independent variable the distance-stiffness function. In some embodiments, the distance-stiffness function is continuous in more than 95% of the value interval of the independent variable the distance-stiffness function. In some embodiments, the distance-stiffness function is continuous in the entire value interval of the independent variable the distance-stiffness function corresponding to the first predetermined region 300. By setting the distance-stiffness function to be continuous, it is possible to make the magnitude of the first feedback force continuous, thereby preventing the operator from having a stuttering sensation during an operation process, and enhancing the operation experience.

In some embodiments, the derivative of the distance-stiffness function is not less than a first predetermined value. The value interval of the derivative of the distance-stiffness function is limited by the first predetermined value, thereby limiting the incremental or decremental nature of the distance-stiffness function. In some embodiments, the first predetermined value may be 0, or some other positive value close to 0. In some embodiments, when the first predetermined value is 0, the derivative of the distance-stiffness function may be 0 or a positive number greater than 0, and the distance-stiffness function may include a constant function or an increasing function. In some embodiments, when the first predetermined value is some other positive values close to 0 (e.g., 0.01, 0.001, etc.), the derivative of the corresponding distance-stiffness function is positive, and the distance-stiffness function may include an increasing function.

In some embodiments, the distance-stiffness function may include a point at where a first order derivative of the distance-stiffness function is discontinuous. There may be two adjacent value intervals of the independent variable of the distance-stiffness function. At a junction point between the two value intervals, the first order derivative of the distance-stiffness function is discontinuous, a change rate of the distance-stiffness function is inconsistent at the junction point, and a tangent slope of the distance-stiffness function at the junction point is inconsistent. For example, at a point F shown in FIG. 6, a segment of the distance-stiffness function at the left of the point F is an increasing function, and the first order derivative of the increasing function at the point F is greater than 0, and a segment of the distance-stiffness function at the right of the point F is a constant function, and the first order derivative of the constant function at the point F is equal to 0. Therefore, the point F is the point at where the first order derivative of the distance stiffness is discontinuous.

In some embodiments, the distance-stiffness function may include two points at where a second order derivative of the distance-stiffness function is discontinuous. That is, there may be two junction points between a plurality of different value intervals, and at the two junction points, the second order derivative of the distance-stiffness function is discontinuous, a change rate of a derivative of the distance-stiffness function is inconsistent, and tangent slopes of a derivative of the distance-stiffness function at the two junction points are inconsistent. In some embodiments, there may be three or more continuous value intervals of the independent variable of the distance-stiffness function, i.e., there may be two or more junction points between the plurality of value intervals of the independent variable, and at the junction points, the second order derivative of the distance-stiffness function is discontinuous. In some embodiments, as shown in FIG. 6, the independent variable of the distance-stiffness function may include three continuous value intervals, and the three value intervals may correspond to the inner layer region 310, the middle layer region 320, the outer layer region 320 of the first predetermined region 300, respectively, a second order derivative of the distance-stiffness function corresponding to the first junction point E of the inner layer region 310 may be different from a second order derivative of the distance-stiffness function corresponding to the first junction point E of the middle layer region 320, and a second order derivative of the distance-stiffness function corresponding to the second junction point F of the middle layer region 320 may be different from a second order derivative of the distance-stiffness function corresponding to the second junction point F of the outer layer region 330. In some embodiments, the independent variable of the distance-stiffness function may include four continuous value intervals, with a first junction point between a first value interval and a second value interval, and a second junction point between the second value interval and a third value interval, and a third junction point between the third value interval and a fourth value interval. The second order derivative of the distance-stiffness function may be discontinuous at the first junction point and at the third junction point, and the second order derivative of the distance-stiffness function may be continuous at the second junction point.

In some embodiments, the distance-stiffness function may include a plurality of value intervals of the independent variable with different functional relationships. In some embodiments, the independent variable of the distance-stiffness function may include a first value interval, a second value interval, and a third value interval with different functional relationships. In some embodiments, a first order derivative of the distance-stiffness function in more than half of the first value interval of the independent variable of the distance-stiffness function is not greater than a second predetermined value; the first order derivative of the distance-stiffness function in more than half of the second value interval of the independent variable of the distance-stiffness function is greater than the second predetermined value; and the first order derivative of the distance-stiffness function in more than half of the third value interval of the independent variable of the distance-stiffness function is not greater than the second predetermined value.

In some embodiments, the first value interval, the second value interval, and the third value interval of the independent variable of the distance-stiffness function may be continuous intervals, and in conjunction with FIG. 3, the first value interval, the second value interval, and the third value interval may correspond to three different spatial regions of the first predetermined region 300. In some embodiments, the first value interval of the independent variable of the distance-stiffness function may correspond to the inner layer region 310 of the first predetermined region 300, and the second value interval of the independent variable of the distance-stiffness function may correspond to the middle layer region 320 of the first predetermined region 300, and the third value interval of the independent variable of the distance-stiffness function may correspond to the outer layer region 330 of the first predetermined region 300. In some embodiments, with reference to FIG. 6, horizontal coordinates of a coordinate system in FIG. 6 are distance equivalents, the first value interval of the distance equivalent may range from 0 to 2/3, which corresponds to boundary points 0 to E of the inner layer region 310 in FIG. 6, i.e., E=2/3; the second value interval of the distance equivalent may range from 2/3 to 5/6, which corresponds to boundary points E to F of the middle layer region 320 in FIG. 6, i.e., F=5/6; and the third value interval of the distance equivalent may range from 5/6 to 1, which corresponds to boundary points F to 1 of the outer layer region 330 in FIG. 6.

In other embodiments, the first value interval, the second value interval, and the third value interval of the independent variable of the distance-stiffness function may also be discontinuous intervals. For example, a value interval of the independent variable of the distance-stiffness function may range from 0 to 1, the first value interval of the independent variable of the distance-stiffness function may range from 0 to 0.32, the second value interval of the independent variable of the distance-stiffness function may range from 0.33 to 0.65, and the third value interval of the independent variable of the distance-stiffness function may range from 0.66 to 1.

In some embodiments, the second predetermined value may be greater than or equal to the first predetermined value. In some embodiments, the second predetermined value may be non-negative. In some embodiments, the first predetermined value may be 0, and a value interval of the second predetermined value may also be 0. In some embodiments, when the second predetermined value is 0, in the inner layer region 310, the first order derivative of the distance-stiffness function in more than half of value interval may be 0, and a distance-stiffness function in the inner layer region 310 may be a constant function; and in the middle layer region 320, the first order derivative of the stiffness function in more than half of a value interval may be greater than 0, and a distance-stiffness function in the middle layer region 320 may be an increasing function; and in the outer layer region 330, the first order derivative of the distance-stiffness function in more than half of a value interval may be 0, and a distance-stiffness function in the outer layer region 330 may be a constant function. In some embodiments, the second predetermined value may also be any positive number greater than 0. Because different spatial regions correspond to different distance-stiffness functions, a change form of the first feedback force subjected by the end tool in different spatial regions may be different. Therefore, a user may determine a spatial region in which the end tool is currently located, i.e., an approximate pose of the end tool currently in the first predetermined region 300 based on a change in the first feedback force, realizing the pose guidance of the end tool.

In some embodiments, the distance-stiffness function may include a plurality of functions, and different value intervals of the independent variable may correspond to different functions. For example, the distance-stiffness function may include a constant function set alternately with an increasing function. In some embodiments, a distance-stiffness function corresponding to the inner layer region 310 may be a first constant stiffness function. A distance-stiffness function corresponding to the middle layer region 320 may be an increasing stiffness function. In some embodiments, the increasing function may include a monotonically increasing quadratic function. In some embodiments, a distance-stiffness function corresponding to the outer layer region 330 may be a second constant stiffness function.

In 520, the first feedback force may be applied to the operator based on a movement direction of the end tool. In some embodiments, operation 520 may be performed by the robotic arm device 110.

In some embodiments, a direction of the first feedback force may always be directed from the end tool toward the predetermined path, thereby prompting the operator of an orientation of the predetermined path relative to the end tool, and preventing the operator from moving the end tool out of the first predetermined region 300. In some embodiments, a resistance to movement of the end tool becomes greater as the end tool moves from the inner layer region 310 into the middle layer region 320. In some embodiments, when the end tool enters the inner layer region 310 from the middle layer region 320, the end tool is not subjected to a resistance as the movement direction of the end tool is toward the predetermined path, i.e., the first feedback force is 0. In some embodiments, the resistance to movement of the end tool becomes greater when a distance equivalent corresponding to a pose of the end tool becomes greater. Referring to FIG. 3, in the case of the inner layer region 310 and the middle layer region 320, for example, since distance-stiffness functions corresponding to the two neighboring spatial regions have a same distance stiffness value at a common boundary, when the end tool travels from one spatial region into another spatial region (e.g., from the inner layer region 310 into the middle layer region 320), the first feedback force subjected by the end tool varies continuously rather than dramatically. This continuously varying first feedback force helps to increase the operator's control of the end tool, thereby increasing the accuracy of the end tool when being dragged by the operator.

In some embodiments, in the first value interval of the distance equivalent (e.g., a range of 0 to 2/3 corresponding to the inner layer region 310), the first feedback force provided by the end tool to the operator ranges from 0 Newtons to 6 Newtons. In some embodiments, the first feedback force provided by the end tool to the operator ranges from 0 Newtons to 4 Newtons in the first value interval of the distance equivalent. In some embodiments, the first feedback force provided by the end tool to the operator ranges from 0 Newtons to 8 Newtons in the first value interval of the distance equivalent. In the second value interval of the distance equivalent (e.g., a range of 2/3 to 5/6 corresponding to the middle layer region 320), the first feedback force provided by the end tool to the operator ranges from 6 Newtons to 208 Newtons. In some embodiments, in the second value interval of the distance equivalent, the first feedback force provided by the end tool to the operator ranges from 4 Newtons to 200 Newtons. In some embodiments, in the second value interval of the distance equivalent, the first feedback force provided by the end tool to the operator ranges from 8 Newtons to 220 Newtons. In the third value interval of the distance equivalent (e.g., a range of 5/6 to 1 corresponding to the outer layer region 330), the first feedback force provided by the end tool to the operator ranges from 208 Newtons to 250 Newtons. In some embodiments, in the third value interval of the distance equivalent, the first feedback force provided by the end tool to the operator ranges from 200 Newtons to 250 Newtons. In some embodiments, in the third value interval of the distance equivalent, the first feedback force provided by the end tool to the operator ranges from 220 Newtons to 250 Newtons.

FIG. 7 is a flowchart illustrating an exemplary process for applying a first feedback torque according to some embodiments of the present disclosure. As shown in FIG. 7, a process 700 may include following operations. In some embodiments, the process 700 may be performed by the surgical robot 100. Operations of process 700 presented below is illustrative. In some embodiments, one or more additional operations not described and/or one or more operations not discussed may be utilized to complete the process.

In 710, a current angle and a current angular stiffness of an end tool may be obtained, and the first feedback torque may be determined based on the current angle and the current angular stiffness. In some embodiments, operation 710 may be performed by the processor 130.

In some embodiments, the current angle of the end tool is an angle between an axis of the end tool and a predetermined path (e.g., a center axis of a tapered region of the first predetermined region 300). In some embodiments, a maximum current angle allowed by the end tool is a cone angle of the tapered region of the first predetermined region 300. When the axis of the end tool is coincident with or parallel to the predetermined path, the current angle is minimum, with an angle value being 0. When the axis of the end tool is parallel to a boundary of the tapered region, the current angle allowed is maximum, with an angle value being half of the cone angle. In some embodiments, the angular stiffness is the ability of the end tool to resist rotation. The current angular stiffness of the end tool is related to the current angle of the end tool. For example, the greater the angle of the end tool with respect to the predetermined path is, the greater the current angular stiffness of the end tool may be.

In some embodiments, a corresponding relationship involves between the current angular stiffness of the end tool and the current angle. In some embodiments, the corresponding relationship may be in tabular form, and an angle value or an angle range may correspond to an angular stiffness value. In other embodiments, the corresponding relationship may also be in the form of a function, the angle value may be used as an independent variable of the function, the angular stiffness value may be used as a dependent variable of the function, and each of the angle values may be used to determine a corresponding angular stiffness value based on the function.

In some embodiments, the corresponding relationship may be pre-stored in the processor 130 or may be entered temporarily. In some embodiments, the operator may view or modify the corresponding relationship.

In some embodiments, the current angular stiffness may be determined based on an angle-stiffness function. In some embodiments, an independent variable of the angle-stiffness function may be the current angle between the end tool and the predetermined path, and the current angular stiffness corresponding to the current angle may be determined based on the angle-stiffness function.

In other embodiments, the independent variable of the angle-stiffness function may be an angular equivalent of a current position of the end tool in the first predetermined region 300 relative to the predetermined path. In some embodiments, the angular equivalent may be a ratio of the current angle corresponding to the current pose of the end tool to a maximum angle, i.e., in a tapered region, the ratio of the current angle corresponding to the current pose of the end tool to half of a cone angle in the tapered region. After determining the current angle corresponding to the current position and angle of the end tool, the angular equivalent corresponding to the angle may be determined; based on the angular equivalent and the angle-stiffness function corresponding to the current angle, the current angular stiffness corresponding to the current angle may be determined.

FIG. 8 is a schematic diagram illustrating an exemplary image of an angle-stiffness function according to some embodiments of the present disclosure. Referring to FIG. 8, horizontal coordinates of a coordinate system shown in FIG. 8 are a current angular equivalent of an end tool, i.e., an image when an independent variable of the angle-stiffness function is an angular equivalent. In some embodiments, a value interval of the independent variable of the angle-stiffness function (e.g., the angular equivalent) may be 0 to 1. In some embodiments, the angle-stiffness function may include a constant function and a monotonically increasing quadratic function. In other embodiments, the angle-stiffness function may include a discrete function. In other embodiments, the angle-stiffness function may include other monotonically increasing functions, such as a cubic function, a segmented function, or the like.

In some embodiments, the independent variable of the angle-stiffness function may have three continuous value intervals, a first angle interval comprising a minimum angle, a third angle interval comprising a maximum angle, and a second angle interval located between the first angle interval and the third angle interval. Correspondingly, the first predetermined region 300 may be divided into three different spatial regions including a first angle region comprising a center axis, a third angle region comprising a boundary of the tapered region, a second angle region located between the first angle region and the third angle region. It should be noted that since the tapered region is axially symmetric, the three angle regions are also axially symmetric.

In some embodiments, the angle-stiffness function may include a segmented function, with different value intervals of the independent variable corresponding to different angle-stiffness functions. For example, the angle-stiffness function may be a constant function set alternately with an increasing function. In some embodiments, an angle-stiffness function corresponding to the first angle interval may be a first constant angle-stiffness function, an angle-stiffness function corresponding to the second angle interval may be an increasing angle-stiffness function, and an angle-stiffness function corresponding to the third angle interval may be a second constant angle-stiffness function. In some embodiments, the angle-stiffness function may also be a non-segmented function in the value interval of the independent variable.

In some embodiments, the angle-stiffness function is continuous in more than half of a value interval of the independent variable of the angle-stiffness function. In some embodiments, the independent variable of the angle-stiffness function may include three continuous value intervals, as shown in FIG. 8. A junction point between the first angle interval and the second angle interval is set as a first junction point G, and a junction point between the second angle interval and the third angle interval is set as a second junction point H. At this time, a value of the angle-stiffness function corresponding to the first junction point G of the first angle interval may and a value of the angle-stiffness function at the first junction point G of the second angle interval are the same, so that the angle-stiffness function may be continuous in the first angle interval and the second angle interval. A value of the angle-stiffness function corresponding to the second junction point H of the second angle interval may and a value of the angle-stiffness function at the second junction point H of the third angle interval are the same, so that that the angle-stiffness function may be continuous in the second angle interval and the third angle interval. In some embodiments, the angle-stiffness function is continuous in more than 60% of the value interval of the independent variable of the angle-stiffness function. In some embodiments, the angle-stiffness function is continuous in more than 80% of the value interval of the independent variable of the angle-stiffness function. In some embodiments, the angle-stiffness function is continuous in more than 90% of the value interval of the independent variable of the angle-stiffness function. In some embodiments, the angle-stiffness function is continuous in more than 95% of the value interval of the independent variable of the angle-stiffness function. In some embodiments, the angle-stiffness function is continuous in the entire value interval of the independent variable of the angle-stiffness function. By setting the angle-stiffness function to be continuous, it is possible to make the magnitude of a first feedback torque continuous, thereby preventing the operator from feeling having a stuttering sensation during an operation process, and enhancing the operation experience.

In some embodiments, the derivative of the angle-stiffness function is not less than a third predetermined value. The value interval of the derivative of the angle-stiffness function is limited by the third predetermined value, thereby limiting the incremental or decremental nature of the angle-stiffness function. In some embodiments, the third predetermined value may be 0, or some other positive value close to 0. In some embodiments, when the third predetermined value is 0, the derivative of a corresponding angle-stiffness function may be 0, or a positive number greater than 0, and the angle-stiffness function may include a constant function or an increasing function. In some embodiments, when the third predetermined value is some other positive value close to 0 (e.g., 0.01, 0.001, etc.), the derivative of the corresponding angle-stiffness function is positive and the angle-stiffness function may include an increasing function.

In some embodiments, the angle-stiffness function may include a point at where a first order derivative of the angle-stiffness function is discontinuous. There may be two adjacent value intervals of the independent variable of the angle-stiffness function. At a junction point between the two value intervals, the first order derivative of the angle-stiffness function is discontinuous, a change rate of the angle-stiffness function is inconsistent, and a tangent slope of the angle-stiffness function is inconsistent. For example, at a point H shown in FIG. 8, a segment of the angle-stiffness function at the left of the point H is an increasing function, and the first order derivative of the increasing function at the H point is greater than 0, and a segment of the angle-stiffness function at the right of the point H is a constant function, and the first order derivative of the constant function at the point H is equal to 0. Therefore, the point H is the point at where the first order derivative of the angle-stiffness function is discontinuous.

In some embodiments, the angle-stiffness function may include two points at where a second order derivative of the angle-stiffness function is discontinuous. That is, there may be two junction points between a plurality of different value intervals, and at the two junction points, a change rate of the second derivative of the angle-stiffness function is inconsistent, and tangents slope of a derivative of the angle-stiffness function at the tow junction points are inconsistent. In some embodiments, the angle-stiffness function may have three or more continuous value intervals of the independent variable of the angle-stiffness function, i.e., there may be two or more junction points between the plurality of value intervals of the independent variable, and at the two or more junction points, the second order derivative of the angle-stiffness function is discontinuous. In some embodiments, as shown in FIG. 8, the independent variable of the angle-stiffness function may include three continuous value intervals: the first angle interval (i.e., the first value interval of the independent variable of the angle-stiffness function), the second angle interval (i.e., the second value interval of the independent variable of the angle-stiffness function) and the third angle interval (i.e., the third value interval of the independent variable of the angle-stiffness function), and a second order derivative of the angle-stiffness function corresponding to the junction point G in the first angle interval may be different from a second order derivative of the angle-stiffness function at the junction point G in the second angle interval, and a second order derivative of the angle-stiffness function corresponding to the junction point H in the second angle interval may be different from a second order derivative of the angle-stiffness function at the junction point H in the third angle interval. In some embodiments, there may be four continuous value intervals of the independent variable of the angle-stiffness function, with a first junction point between a first value interval and a second value interval, and a second junction point between the second value interval and a third value interval, and a third junction point between the third value interval and a fourth value interval. The second order derivative of the angle-stiffness function may be discontinuous at the first junction point and at the third junction point, and the second order derivative of the angle-stiffness function may be continuous at the second junction point.

In some embodiments, the angle-stiffness function may include a plurality of value intervals of the independent variable with different functional relationships. In some embodiments, the independent variable of the angle-stiffness function may include a first value interval, a second value interval, and a third value interval with different functional relationships. In some embodiments, a plurality of value intervals with different functional relationships of the angle-stiffness function may be continuous value intervals. In other embodiments, the plurality of value intervals with different functional relationships of the angle-stiffness function may be discontinuous value intervals. In some embodiments, a first order derivative of the angle-stiffness function in more than half of the first value interval of the independent variable of the angle-stiffness function (the first angle interval) is not greater than a fourth predetermined value; the first order derivative of the angle-stiffness function in more than half of the second value interval (the second angle interval) of the independent variable of the angle-stiffness function is greater than the fourth predetermined value; the first order derivative of the angle-stiffness function in more than half of the third value interval (the third angle interval) of the independent variable of the angle-stiffness function is not greater than the fourth predetermined value.

In some embodiments, the fourth predetermined value may be greater than or equal to the third predetermined value. In some embodiments, the fourth predetermined value may be non-negative. In some embodiments, the third predetermined value may be 0, and a range of values of the fourth predetermined value may also be 0. In some embodiments, when the fourth predetermined value is 0, in the first angle interval, the first order derivative of the angle-stiffness function in more than half of a value interval may be 0, and the angle-stiffness function in the first angle interval may be a constant function; in the second angle interval, the first order derivative in more than half of a value interval of the angle-stiffness function may be greater than 0, and the angle-stiffness function in the second angle interval may be an increasing quadratic function; in the third angle interval, the first order derivative of the angle-stiffness function in more than half of a value interval of the angle-stiffness function may be 0, and the angle-stiffness function in the third angle interval may be a constant function. Since angle-stiffness functions corresponding to different angle intervals are different, a change form of the first feedback torque subjected by the end tool in spatial regions corresponding to different angle intervals may be different. Therefore, a user may determine a spatial region in which the end tool is currently located according to the change form of the first feedback torque, i.e., an approximate pose of the end tool in the first predetermined region 300, henceforth realizing the pose guidance of the end tool.

In some embodiments, the independent variable of the angle-stiffness function may be an angular equivalent of a current pose of the end tool in the first predetermined region 300 relative to a predetermined path. At this point, referring to FIG. 8, horizontal coordinates of a coordinate system in FIG. 8 are angular equivalents, and the first value interval of the angular equivalent may range from 0 to 2/3, which corresponds to boundary points 0 to G of the first angle interval in FIG. 8, G=2/3; the second value interval of the angular equivalent may range from 2/3 to 5/6, which corresponds to boundary points G to H of the second angle interval in FIG. 8, H=5/6; and the third value interval of the angular equivalent may range from 5/6 to 1, which corresponds to boundary points H to 1 of the third angle interval in FIG. 8.

In720, the first feedback torque may be applied to the operator based on a movement direction of the end tool. In some embodiments, operation 720 may be performed by the robotic arm device 110.

In some embodiments, the direction of the first feedback torque may always be in a rotation direction of the end tool toward the predetermined path, thereby prompting the operator to an orientation the predetermined path relative to a pose of the end tool, and preventing the operator from rotating the end tool outside the first predetermined region 300. In some embodiments, when the end tool is rotated from an angle region with a smaller angle value to an angle region with a larger angle value, a resistance to rotation of the end tool becomes greater. In some embodiments, when the end tool is rotated from the angle region with the larger angle value to the angle region with the smaller angle value, the end tool is not subjected to a hindering toque as the rotation direction the end tool is directed toward the predetermined path , i.e., the first feedback torque is 0. In some embodiments, since three value intervals of the independent variable of the angle-stiffness function are continuous, angle-stiffness functions corresponding to two adjacent value intervals at a common boundary have the same angular stiffness value, so the first feedback torque subjected by the end tool varies continuously rather than dramatically when an angle between the end tool and the predetermined path changes moves from one value interval to another. This continuously varying first feedback torque helps to improve the operator's control of the end tool, thereby improving the accuracy of the movement of the end tool when being dragged by the user.

In other embodiments, the direction of the first feedback torque may be determined based on the movement direction of the end tool. For example, the direction of the first feedback torque may be opposite to the rotation direction of the end tool. The first feedback torque may initiate a blocking effect when the end tool is rotated in a direction away from the predetermined path, and the first feedback torque may initiate a boosting effect when the end tool is rotated in a direction closer to the predetermined path, thereby prompting the operator the orientation of the predetermined path relative to the position of the end tool.

In some embodiments, since different value intervals correspond to different angle-stiffness functions, the first feedback torque is determined based on a product of a current angle between the end tool and the predetermined path and an angular stiffness value corresponding to the current angle, and the angular stiffness value is determined by the current angle and an angle-stiffness function corresponding to the current angle, thus the angle-stiffness function determines a change form of the first feedback torque. Therefore, the operator may determine a value interval in which the end tool is located based on the change form of the first feedback torque, i.e., determine a range of angles between the end tool and the predetermined path, which can help to improve the user's control over the pose of the end tool, thereby improving the speed and accuracy of orthopedic surgery.

In some embodiments, in the first value interval of the angular equivalent, the first feedback torque provided by the end tool to the operator ranges from 0 Newton-meter to 17 Newton-meter. In some embodiments, in the first value interval of the angular equivalent, the first feedback torque provided by the end tool to the operator ranges from 0 Newton-meter to 14 Newton-meter. In some embodiments, in the first value interval of the angular equivalent, the first feedback torque provided by the end tool to the operator ranges from 0 Newton-meter to 20 Newton-meter. In some embodiments, in the second value interval of the angular equivalent, the first feedback torque provided by the end tool to the operator ranges from 17 Newton-meter to 65 Newton-meter. In some embodiments, in the second value interval of the angular equivalent, the first feedback torque provided by the end tool to the operator ranges from 14 Newton-meter to 62 Newton-meter. In some embodiments, in the second value interval of the angular equivalent, the first feedback torque provided by the end tool to the operator ranges from 20 Newton-meter to 68 Newton-meter. In some embodiments, in the third value interval of the angular equivalent, the first feedback torque provided by the end tool to the operator ranges from 65 Newton-meter to 78.5 Newton-meter. In some embodiments, in the third value interval of the angular equivalent, the first feedback torque provided by the end tool to the operator ranges from 62 Newton-meter to 78.5 Newton-meter. In some embodiments, in the third value interval of the angular equivalent, the first feedback torque provided by the end tool to the operator ranges from 68 Newton-meter to 78.5 Newton-meter.

FIG. 9 is a flowchart illustrating an exemplary process for applying a second feedback force according to some embodiments of the present disclosure. As shown in FIG. 9, a process 900 may include following operations. In some embodiments, the process 900 may be performed by the surgical robot 100. Operations of the process 900 presented below is illustrative. In some embodiments, one or more additional operations not described and/or one or more operations not discussed may be utilized to complete the process.

In 910, a current speed and a current damping of an end tool may be obtained. In some embodiments, operation 910 may be performed by the processor 130.

In some embodiments, a corresponding damping function may be determined by the current speed of the end tool, thereby determining the current damping corresponding to the current speed. In some embodiments, an allowable speed of the end tool does not exceed a predetermined speed, so as not to avoid an accident due to excessive movement of the end tool. For example, this may injure a wound in an opening region of a surgical object, which may affect surgical result, or the like. In some embodiments, the predetermined speed is a value of a maximum speed that the end tool is allowed to reach. The predetermined speed may be determined by an operator on a case-by-case basis.

In some embodiments, a corresponding relationship involves between the current damping and the current speed of the end tool. In some embodiments, the corresponding relationship may be in tabular form, and a speed value or a speed range may correspond to a damping value. In other embodiments, the corresponding relationship may also be in the form of a function, where the speed value may be used as an independent variable of the function, the damping value may be used as a dependent variable of the function, and each of the speed value may be used to determine a corresponding damping value based on the function.

In some embodiments, the corresponding relationship may be pre-stored in the processor 130 or may be entered temporarily. In some embodiments, the operator may view or modify the corresponding relationship.

In some embodiments, the current damping may be determined based on a damping function. In some embodiments, an independent variable of the damping function may be a current speed of the end tool, and the current damping corresponding to the speed may be determined by the damping function, e.g., a damping magnitude corresponding to a magnitude of the speed and a damping direction corresponding to a direction of the speed. In some embodiments, a value interval of the current speed may range from a minimum allowable speed to a maximum allowable speed. In some embodiments, the minimum allowable speed and the maximum allowable speed may be set by the operator as appropriate. In some embodiments, the minimum allowable speed may be 0.

FIG. 10 is a schematic diagram illustrating an exemplary image of a damping function according to some embodiments of the present disclosure. Referring to FIG. 10, horizontal coordinates of a coordinate system shown in FIG. 10 are a current speed of an end tool, i.e., an independent variable of a damping function is a speed. In some embodiments, the damping function may be a segmented function in a value interval of the independent variable of the damping function. In other embodiments, the damping function may also be a non-segmented function in the value interval of the independent variable of the damping function. In some embodiments, the damping function may include a constant function and a monotonically increasing quadratic function. In other embodiments, the damping function may include a discrete function. In other embodiments, the damping function may include other monotonically increasing functions, such as a cubic function, a segmented function, or the like.

In some embodiments, the independent variable of the damping function is the current speed of the end tool. In some embodiments, the damping function may include a segmented function (i.e., a plurality of functions), with different value intervals of the independent variable corresponding to different functions. For example, the damping function may be a constant function set alternately with an increasing function. In some embodiments, a damping function corresponding to a first speed interval may be a first constant damping function, a damping function corresponding to a second speed interval may be an increasing damping function, and a damping function corresponding to a third speed interval may be a second constant damping function. In some embodiments, the second constant damping function may include a damping function corresponding to a maximum allowable speed of the end tool.

In some embodiments, the damping function may also be expressed as a function. In some embodiments, the damping function is continuous in more than half of a value interval of the independent variable of the damping function. In some embodiments, the damping function may include a plurality of value intervals of independent variable with different functional relationships. In some embodiments, the plurality of value intervals of the independent variable with different functional relationships of the damping function may include a first value interval (also referred to as a first speed interval), a second value interval (also referred to as a second speed interval), and a third value interval (also referred to as a third speed interval). In some embodiments, the first value interval, the second value interval, and the third value interval of the independent variable of the damping function may be three continuous value intervals, as shown in FIG. 10. A junction point between the first speed interval and the second speed interval is set to be a first junction point J, and a junction point between the second speed interval and the third speed interval is set to be a second junction point K. A value of the damping function corresponding to the first junction point J of the first speed and a value of the damping function at the first junction point J of the second speed may be the same, so that the damping function may be continuous in the first speed interval and the second speed interval. The value of the damping function corresponding to the second junction K of the second speed and a value of the damping function at the second junction K of the third speed may be the same, so that the damping function may be continuous in the second speed interval and the third speed interval. In some embodiments, the damping function is continuous in more than 60% of a value interval of the independent variable of the damping function. In some embodiments, the damping function is continuous in more than 80% of the value interval of the independent variable of the damping function. In some embodiments, the damping function is continuous in more than 90% of the value interval of the independent variable of the damping function. In some embodiments, the damping function is continuous in more than 95% of the value interval of the independent variable of the damping function. In some embodiments, the damping function is continuous in an entire value interval of an independent variable corresponding to the first predetermined region 300. By setting the damping function to be continuous, it is possible to make the magnitude of a first feedback force continuous, thereby preventing an operator from having a stuttering sensation during an operation process and enhancing the operation experience. In other embodiments, the first value interval, the second value interval, and the third value interval of the independent variable of the damping function may also be discontinuous value intervals.

In some embodiments, the derivative of the damping function is not less than a fifth predetermined value. The value interval of the derivative of the damping function is limited by the fifth predetermined value, thereby limiting the incremental or decremental nature of the damping function. In some embodiments, the fifth predetermined value may be 0, or some other positive value close to 0. In some embodiments, when the fifth predetermined value is 0, the derivative of a corresponding damping function may be 0, or a positive number greater than 0, and the damping function may include a constant function or an increasing function. In some embodiments, when the fifth predetermined value is some other positive value near 0 (e.g., 0.01, 0.001, etc.), the derivative of a corresponding damping function is a positive number, and the damping function may include an increasing function.

In some embodiments, the damping function may include a point at where a first order derivative of the damping function is discontinuous. That is, there may be two adjacent value intervals of the independent variable of the damping function, where a first order derivative of the damping function is discontinuous at a junction point between the two value intervals. At the junction point, a change rate of the damping function is inconsistent, and a tangent slope of the damping function at the junction point is inconsistent. For example, at the point K shown in FIG. 10, a segment of the damping function at the left of the point K is an increasing function, and the first order derivative of the increasing function at the point K is greater than 0; and a segment of the damping function at the right of the point K is a constant function, and the first order derivative of the constant function at the point K is equal to 0. Therefore, the point K is the point at where the first order derivative of the damping function is discontinuous.

In some embodiments, the damping function may include two points at where a second order derivative of the damping function is discontinuous. That is, there may be two junction points between a plurality of different value intervals at which the second order derivative of the damping function is discontinuous, a change rate of a derivative of the damping function is inconsistent, and tangent slopes of a derivative of the damping function at the two junction points are inconsistent. In some embodiments, the damping function may include three continuous value intervals of the independent variable of the damping function, i.e., there may be two junction points between the plurality of value intervals of the independent variable at which the second order derivative of the damping function is discontinuous. In some embodiments, as shown in FIG. 10, the independent variable of the damping function may include three continuous value intervals, and a second order derivative of a damping function corresponding to a first junction point J in the first speed interval may be different from a second order derivative of a damping function corresponding to the first junction point J in the second speed interval, and a second order derivative of a damping function corresponding to a second junction point K in the second speed interval may be different from a second order derivative of a damping function corresponding to the second junction point K in the third speed interval. In some embodiments, the damping function may have four continuous value intervals of the independent variable, a junction point between the first value interval and the second value interval is a first junction point, a junction point between the second value interval and the third value interval is a second junction point, and a junction point between the third value interval and the fourth value interval is a third junction point. The second order derivative of the damping function may be discontinuous at the first junction point and at the third junction point, and the second order derivative of the damping function may be continuous at the second junction point.

Referring to FIG. 10, in some embodiments, a first order derivative in more than half of a first value interval of the damping function is not greater than a sixth predetermined value of the damping function; a first order derivative in more than half of a second value interval of the damping function is greater than the sixth predetermined value of the damping function; a first order derivative in more than half of a third value interval of the damping function is not greater than the sixth predetermined value of the damping function.

In some embodiments, the sixth predetermined value may be greater than or equal to the fifth predetermined value. In some embodiments, the sixth predetermined value may be non-negative. In some embodiments, the fifth predetermined value may be 0, and a value interval of the sixth predetermined value may also be 0. In some embodiments, the sixth predetermined value may also be any positive number greater than 0. In some embodiments, the first order derivative of the damping function in more than half of the first speed interval may be 0, at which point a damping function in the first speed interval may be a constant function; the first order derivative of the damping function in more than half of the second speed interval may be greater than 0, at which time a damping function in the second speed interval may be an increasing quadratic function; the first order derivative of the damping function in more than half of the third speed interval may be 0, at which time the damping function in the third speed interval may be a constant function.

In some embodiments, the damping function may also be determined based on a biometric feature of an object. In some embodiments, the biometric feature of the object may include human features such as fat, joint fluid, and so forth, and the damping function may be the same as or similar to a damping of the fat, joint fluid, and so forth.

In 920, a second feedback force based on a current speed and a current damping may be determined. In some embodiments, operation 920 may be performed by the processor 130.

In some embodiments, a product of the current speed and a current damping corresponding to the current speed may be used as a magnitude of the second feedback force. In some embodiments, a direction of the second feedback force may be opposite to a direction of the current speed, i.e., when the operator controls the end tool to move, the end tool may provide the operator with a second feedback force whose direction is opposite to a movement direction of the end tool. In other embodiments, the direction of the second feedback force may be toward a predetermined path, i.e., when the operator controls the end tool to move away from the predetermined path, the end tool may provide the operator with a second feedback force whose direction is opposite to the movement direction of the end tool, and when the operator controls the end tool to move toward the predetermined path, the end tool may not provide the second feedback force. In other embodiments, the direction of the second feedback force may be away from the predetermined path, i.e., when the operator controls the end tool to move toward the predetermined path, the end tool may provide a second feedback force whose direction is opposite to the movement direction of the end tool, and when the operator controls the end tool to move away from the predetermined path, the end tool may provide only the first feedback force and not the second feedback force.

In some embodiments, because damping functions corresponding to two adjacent speed intervals have a same damping value at a common boundary, when the end tool moves from one speed interval to another, a second feedback force subjected by the end tool varies continuously rather than dramatically. Such continuously varying second feedback force helps to increase the operator's control of the end tool, thereby increasing the accuracy of the end tool when dragged by the operator.

In some embodiments, since different speed intervals correspond to different damping functions (or different functional relationships), the second feedback force is determined based on the product of the current speed of the end tool and the current damping corresponding to the current speed, and the damping is determined by a product of the current speed of the end tool and a damping function corresponding to the current speed, thus the damping function determines a change form of the second feedback force. The operator may therefore determine a speed range of the end tool based on the change form of the second feedback force.

It should be noted that when the end tool provides both the first feedback force and the second feedback force to the operator, a total feedback force subjected by the operator is a vector sum of the first feedback force and the second feedback force. Therefore, a change form of the total feedback force is a vector combination of a change form of the first feedback force and a change form of the second feedback force, so the operator may determine a vector combination of a change form of a first feedback force and a change form of a second feedback force corresponding to a change form of a total feedback force currently, and then determine a spatial region in which the end tool is located and a speed range based on sensation corresponding to the vector combination.

FIG. 11 is a flowchart illustrating an exemplary process for applying a second feedback torque according to some embodiments of the present disclosure. As shown in FIG. 11, a process 1100 may include following operations. In some embodiments, the process 1100 may be performed by the surgical robot 100. Operational intent of the process 1100 presented below is illustrative. In some embodiments, the process may be accomplished utilizing one or more additional operations not described and/or one or more operations not discussed.

In 1110, a current angular speed of an end tool and a current rotational damping corresponding to the current angular speed may be obtained. In some embodiments, operation 1110 may be performed by the processor 130.

In some embodiments, a corresponding rotational damping function may be determined based on the current angular speed of the end tool, thereby determining the current rotational damping corresponding to the current angular speed. In some embodiments, an allowable angular speed of the end tool does not exceed a predetermined angular speed, so as to prevent an accident due to the end tool moving too fast, for example, the end tool moving too fast may injure a wound in an open area of a surgical object, affecting a surgical result, etc. In some embodiments, the predetermined angular speed is a maximum angular speed value that the end tool is allowed to reach. The predetermined angular speed may be determined by an operator on a case-by-case basis.

In some embodiments, a corresponding relationship may involve between the current rotational damping and the current angular speed of the end tool. In some embodiments, the corresponding relationship may be in a tabular form, and an angular speed value or a range of angular speeds may correspond to a rotational damping value. In other embodiments, the corresponding relationship may also be in the form of a function, where the angular speed value may serve as an independent variable of the function, the rotational damping value may serve as a dependent variable of the function, and each of the angular speed value may be used to determine a corresponding rotational damping value based on the function.

In some embodiments, the corresponding relationship may be pre-stored in the processor 130 or may be entered temporarily. In some embodiments, the operator may view or modify the corresponding relationship.

In some embodiments, the current rotational damping may be determined based on a rotational damping function. In some embodiments, an independent variable of the rotational damping function may be a current angular speed of the end tool, and a current rotational damping corresponding to the current angular speed may be determined based on the rotational damping function.

In some embodiments, the rotational damping function may include a constant function and a monotonically increasing quadratic function. In other embodiments, the rotational damping function may also include a discrete function. In other embodiments, the rotational damping function may also include other monotonically increasing functions, such as a cubic function, a segmented function, or the like.

In some embodiments, the independent variable of the rotational damping function takes the current angular speed of the end tool. In some embodiments, the current angular speed may take on a value interval ranging from a minimum allowable angular speed to a maximum allowable angular speed. In some embodiments, the minimum allowable angular speed and the maximum allowable angular speed may be set by the operator as appropriate. In some embodiments, the minimum allowable angular speed may be 0. In some embodiments, the rotational damping function may include a segmented function, with different value intervals of the independent variable corresponding to different rotational damping functions. For example, the rotational damping function may be a constant function set alternately with an increasing function. In some embodiments, a rotational damping function corresponding to a first value interval may be a first constant rotational damping function, a rotational damping function corresponding to a second value interval may be an incremental rotational damping function, and a rotational damping function corresponding to a third value interval may be a second constant rotational damping function.

In some embodiments, the rotational damping function is continuous in more than half of the value interval of the independent variable of the rotational damping function. In some embodiments, the rotational damping function is continuous in more than 60% of the value interval of the independent variable of the rotational damping function. In some embodiments, the rotational damping function is continuous in more than 80% of the value interval of the independent variable of the rotational damping function. In some embodiments, the rotational damping function is continuous in more than 90% of the value interval of the independent variable of the rotational damping function. In some embodiments, the rotational damping function is continuous in more than 95% of the value interval of the independent variable of the rotational damping function. In some embodiments, the rotational damping function is continuous in the entire value interval of the independent variable of the rotational damping function. In some embodiments, the independent variable of the rotational damping function may include three continuous value intervals. A junction point between a first angular speed interval and a second angular speed interval is set to be a first junction point, and a junction point between the second angular speed interval and a third angular speed interval is set to be a second junction point. A rotational damping function value corresponding to a first junction point of the first angular speed interval and a rotational damping function value corresponding to a first junction point of the second angular speed interval may be the same, so that the rotational damping function may be continuous in the first angular speed interval and the second angular speed interval. A rotational damping function value corresponding to a second junction point of the second angular speed interval and a rotational damping function value corresponding to a second junction point of the third angular speed interval may be the same, so that the rotational damping function may be continuous in the second angular speed interval and the third angular speed interval.

In some embodiments, a derivative of the rotational damping function may be 0 or a positive number greater than 0.

In some embodiments, the rotational damping function may include a point at where the first order derivative of the rotational damping function is discontinuous. That is, there may be two adjacent value intervals of the independent variable, and at a junction point between the two value intervals, the first order derivative of the rotational damping function is discontinuous, and a change rate of the rotational damping function at this junction point is inconsistent, and a tangent slope of the rotational damping function at this junction point is inconsistent.

In some embodiments, the rotational damping function may include two points at where a second order derivative of the rotational damping function is discontinuous. That is, there may be two junction points between a plurality of different value intervals, and at the two junction points, the second order derivative of the rotational damping function is discontinuous, a change rate of a derivative of the rotational damping function is inconsistent, and tangent slopes of the derivative of the rotational damping function are inconsistent.

In some embodiments, the rotational damping function may also be determined based on a biometric feature of an object. In some embodiments, the biometric feature of the object may include human features such as fat, joint fluid, etc., and the rotational damping function may be the same as or similar to a rotational damping of the fat, joint fluid, etc.

In 1120, a second feedback torque based on the current speed and current damping may be determined. In some embodiments, operation 1120 may be performed by the processor 130.

In some embodiments, the second feedback torque may be determined by the current angular speed and the current rotational damping corresponding to the current angular speed.

In some embodiments, since the three value intervals of the independent variable of the rotational damping function are continuous, a rotational damping function corresponding to two adjacent value intervals has a same rotational damping value at a common boundary, thus when an angle between the end tool and a predetermined path moves from one value interval to another value interval, a second feedback torque subjected by the end tool changes continuously rather than dramatically. Such continuously varying second feedback torque helps to increase the operator's control of the end tool, thereby increasing the accuracy of the end tool while being dragged by the operator.

During a surgical operation, in order to enable the end tool to precisely operate on the surgical object, it is generally necessary to keep a center axis of the end tool to coincide or substantially coincide with a center axis of an operation region (e.g., the first predetermined region 300, the second predetermined region 400, etc.) However, before or during the operation, the object may move, thereby changing a pose of the object, so that the center axis of the end tool may no longer coincide or substantially coincide with the center axis of the operation region, which affects the surgical effect. At this time, it is necessary to adjust the end tool's pose according to a change in the pose of the object, so as to facilitate the surgical operation. It should be noted that the two axes described in the present disclosure as "substantially coincide" means that, in a range of the operation region, a maximum distance between the two axes does not exceed 0.1 mm.

FIG. 12 is a flowchart illustrating an exemplary process for actively adjusting a pose of an end tool according to some embodiments of the present disclosure. As shown in FIG. 12, a process 1200 may include following operations. In some embodiments, the process 1200 may be performed by the surgical robot 100. Operational intent of the process 1200 presented below is illustrative. In some embodiments, the process may be accomplished utilizing one or more additional operations not described and/or one or more operations not discussed.

In some embodiments, the end tool may perform a surgical operation in the second predetermined region 400, the second predetermined region 400 is an actively following region. After a surgical object moves, the second predetermined region 400 follows the object to move, and the second predetermined region 400 remains relatively stationary with the object. At this time, the robotic arm device 110 may actively control the end tool to generate a corresponding movement so that a position of the end tool remains unchanged in the second predetermined region 400, i.e., the position of the end tool remains unchanged in the second predetermined region 400, so that a relative position of the end tool and the object remains unchanged, which facilitates the surgery and reduces the possibility of accidents in the surgery.

In 1210, a first initial pose of the end tool and a pose offset of the object may be obtained. In some embodiments, operation 1210 may be performed by the processor 130.

In some embodiments, the first initial pose of the end tool refers to a pose of the end tool in the second predetermined region 400 before the object moves, and the pose of the end tool may include a distance between a reference point of the end tool with respect to a target position of the object, an angle between a center axis of the end tool and an axis of the object with the target pose, or the like. In some embodiments, the pose offset of the object refers to an amount of change in a pose of the object before and after the object moves. In some embodiments, the first initial pose of the end tool may be determined by the positioning device 120.

In some embodiments, the positioning device 120 may include a first optical array disposed on a base for supporting the robotic arm device 110, a second optical array disposed on the robotic arm device 110, and a third optical array disposed on the target object (e.g., an operation region). The second optical array maintains a constant pose corresponding relationship (the end tool) (i.e., the second optical array moves in synchronization with the end tool, and a relative position between the two remains unchanged) with the robotic arm device 110, and the third optical array maintains a constant pose corresponding relationship with the second predetermined region 400 (the object) (i.e., the second optical array moves in synchronization with the end tool, and a relative position between the two remains unchanged). In some embodiments, the first optical array remains relatively fixed to the base and may serve as a datum reference point for a reference coordinate system. The reference coordinate system is a coordinate system that is unaffected by the movement of the end tool and the object, i.e., the reference coordinate system may remain immobile when the end tool and the object move, and the end tool and the object move within the reference coordinate system. In other embodiments, the first optical array that serves as the datum reference point for the reference coordinate system may also be located on one of other parts of the surgical robot 100 whose position is unchanged (e.g., a wheel, etc.) or an object whose positions are unchanged in a surrounding environment (e.g., a bed, etc.). In some embodiments, the first optical array may serve as the reference datum point for the reference coordinate system. More descriptions about the first optical array, the second optical array, the third optical array, and the base can be found with reference to FIG. 20 and its related descriptions, which will not be repeated here.

In some embodiments, the first initial pose of the end tool may be determined as $^{s}T_{marker2\_static}$ by the second optical array.

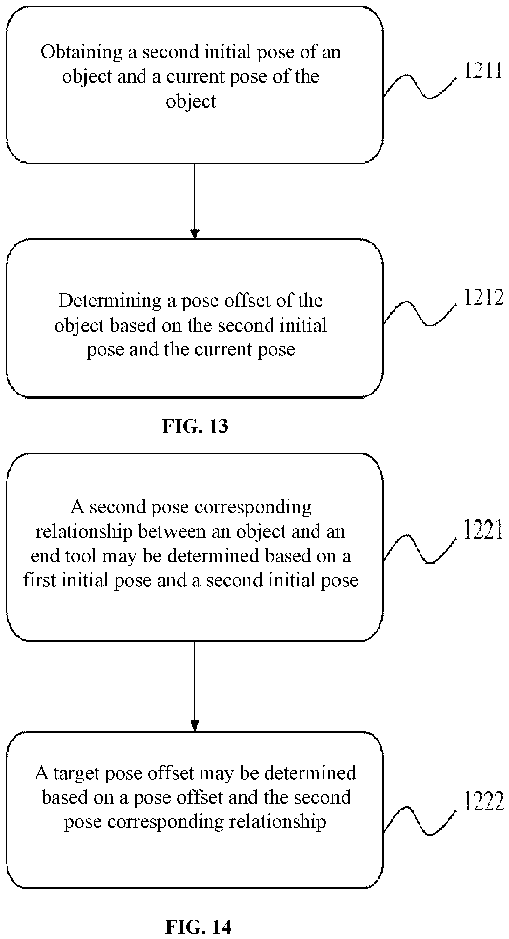

FIG. 13 is a flowchart illustrating an exemplary process for determining a pose offset of an object according to some embodiments of the present disclosure. Referring to FIG. 13, in some embodiments, operation 1210 may determine the pose offset of the object by following sub operations including operation 1211: obtaining a second initial pose of the object and a current pose of the object; and operation 1212: determining the pose offset of the object based on the second initial pose and the current pose.

In some embodiments, the second initial pose of the object refers to a pose of the object before the pose of the object changes. The current pose of the object refers to a current pose of the object after the object moves. The pose offset of the object may be understood as a movement amount of the object from the second initial pose to the current pose.

In some embodiments, the second initial pose of the object may be determined by a third optical array as $^sT_{marker3\_static}$, and the current pose of the object may be determined by the third optical array as $^sT_{marker3\_dynamic}$. In some embodiments, the current pose of the object may be obtained by the third optical array when a controller detects a pose following signal and detects a change in the current pose of the object compared to the second initial pose at the same time. In some embodiments, the third optical array obtains the current pose of the object at a frequency greater than or equal to 250 times/second to satisfy a frequency requirement for correcting a pose of the end tool (e.g., greater than or equal to 250 times/second ). In some embodiments, the pose offset of the object may be determined based on a following equation (1):

$$^{marker3\_static}T_{maeker3\_dynamic} \cdot \quad ^{marker3\_static}T_{maeker3\_dynamic} = \mathrm{Inverse}(^sT_{marker3\_static}) \times {}^sT_{marker3\_dynamic} \quad (1)$$

where, $\mathrm{Inverse}(^sT_{marker3\_static})$ is an inverse matrix of the second initial pose $^sT_{marker3\_static}$ of the object.

In 1220, a target pose offset of the end tool may be determined based on the first initial pose and the pose offset. In some embodiments, operation 1220 may be performed by the processor 130.

In some embodiments, based on the first initial pose $^sT_{marker2\_static}$ of the end tool and the pose offset $^{marker3\_static}T_{maeker3\_dynamic}$ of the object, the target pose offset of the end tool may be determined by a following equation (2):

$$^{tool_{static}}T_{tool_{dynamic}} = \mathrm{Inverse}\left(^{object}T_{tool}\right) * {}^{marker3\_static}T_{marker3\_dynamic} * {}^{object}T_{tool} \quad (2)$$

where, $^{object}T_{tool}$ is a pose change relationship between the object and the end tool before the object moves, i.e., a second pose corresponding relationship of the first initial pose of the end tool relative to the second initial pose of the object. $\mathrm{Inverse}(^{object}T_{tool})$ is an inverse matrix of $^{object}T_{tool}$. More descriptions about the second pose corresponding relationship $^{object}T_{tool}$ can be found in FIG. 14 and its related description.

FIG. 14 is a flowchart illustrating an exemplary process for determining a target pose offset of an end tool according to some embodiments of the present disclosure. Referring to FIG. 14, in some embodiments, operation 1220 may determine the target pose offset of the end tool by following sub-operations. In operation 1221, a second pose corresponding relationship between an object and the end tool may be determined based on a first initial pose and a second initial pose. In 1222, the target pose offset may be determined based on a pose offset and the second pose corresponding relationship.

In some embodiments, the second pose corresponding relationship of the first initial pose of the end tool relative to the second initial pose of the object $^{object}T_{tool}$ may refer to a relative relationship that needs to be maintained between a pose of the end tool and a pose of the object.

In some embodiments, the second pose corresponding relationship of the first initial pose of the end tool relative to the second initial pose of the object $^{object}T_{tool}$ may be determined by equation (3):

$$^{object}T_{tool} = \mathrm{Inverse}(^sT_{marker3\_static}) * {}^sT_{marker2\_static} \quad (3)$$

Where, $^sT_{marker3\_static}$ is the second initial pose of the object, $\mathrm{Inverse}(^sT_{marker3\_static})$ is an inverse matrix of $^sT_{marker3\_static}$, and $^sT_{marker3\_static}$ is the first initial pose of the end tool.

In some embodiments, based on the pose offset and the second pose corresponding relationship, the target pose offset may be determined by the above equation (2). In some embodiments, the second pose corresponding relationship may be determined and recorded when an operator inputs a recording signal. In some embodiments, the operator may input the recording signal multiple times to record data of a plurality of sets of pose corresponding relationships between the object and the end tool. In some embodiments, when there is a need to control the end tool to actively follow the object, latest recorded data of a pose corresponding relationship between the object and the end tool may be used as the second pose corresponding relationship.

Since the target pose offset of the end tool determined based on the pose offset and the second pose corresponding relationship is a movement amount required for the end tool to be adjusted from the first initial pose to a target pose, by controlling the end tool to move based on the target pose offset, it is possible to restore a pose corresponding relationship between the end tool and the object (the second predetermined region 400) to a pose corresponding relationship between the end tool and the object before the movement of the object when the end tool completes the target pose offset.

In 1230, the end tool may be adjusted to the target pose based on the target pose offset to control the end tool to actively follow the movement of the object. In some embodiments, operation 1230 may be performed by the processor 130.

In some embodiments, after determining the target pose offset, the end tool may be controlled to move to the target pose by moving according to a corresponding target pose offset, thereby causing the end tool to actively follow the object, so as to keep a relative position between the end tool and the object constant, which is conducive to a subsequent surgery.

Since the target pose offset corresponds to the movement amount of the end tool, and the movement of the end tool is driven by the robotic arm device 110, i.e., the processor 130 drives the movement of the end tool by controlling the movement of the robotic arm device 110. Therefore, in some embodiments, a pose adjustment amount may be converted into a datum coordinate system based on a conversion relationship between a coordinate system corresponding to the end tool and a reference coordinate system, thereby determining a pose change amount of the robotic arm device 110 carrying the end tool arm in the datum coordinate system, and controlling the robotic arm device 110 to drive the end tool to move based on the pose change amount, so that when the robotic arm device 110 completes the movement according to the pose change amount, the end tool completes the movement of the target pose offset and reaches the target pose, and at the same time, the pose corresponding relationship between the end tool and the object is restored to the second pose corresponding relationship.

In some embodiments, the process 1200 may further include obtaining a first pose corresponding relationship between the end tool and the reference coordinate system. At this point, operation 1230 may include determining the target pose based on the first pose corresponding relationship and the target pose offset.

In some embodiments, the first pose corresponding relationship between the end tool and the reference coordinate system may be understood as a changing relationship of a reference datum in the reference coordinate system to the first initial pose of the end tool prior to movement of the object. In some embodiments, the first pose corresponding relationship between the end tool and the reference coordinate system may be denoted as $^{B}T_{Tool\_static}$. In some embodiments, the first pose corresponding relationship $^{B}T_{Tool\_static}$ may be derived from the positive kinematics of individual joint positions of the robotic arm device 110 in the surgical robot 100.

In some embodiments, the pose change amount of the robotic arm device 110 may be determined by equation (4):

$$^{B}T_{tool\_dynamic} = {^{B}T_{Tool\_static}} * {^{Tool\_static}T_{tool\_dynamic}} \quad (4)$$

Where, $^{B}T_{tool\_dynamic}$ actually includes $^{B}T_{tool}$ and $^{tool}T_{marker2}$. $^{B}T_{tool}$ is an actual control amount of the robotic arm device 110. $^{tool}T_{marker2}$ is used to limit a movement corresponding relationship between the robotic arm device 110 and the end tool. Because there are a variety of ways in which the robotic arm device 110 drives the movement of the end tool, there is a need to uniquely limit the way in which the robotic arm device 110 drives the movement of the end tool through the movement corresponding relationship, thereby determining the movement of the end tool by uniquely determining the movement of the robotic arm device 110, henceforth realizing any desired pose adjustment by the end tool driven by the robotic arm device 110. It should be noted that $^{tool}T_{marker2}$ may be calibrated in advance, such as hand-eye calibration, etc.

In some embodiments, a controller or processor outputs a pose following completion signal in response to detecting that the end tool completes the movement of a pose adjustment. The pose following completion signal may be output by a blinking light provided on a robotic arm or by an output device connected to the controller or processor, and this embodiment does not limit a specific form of the pose following completion signal.

In some embodiments, the controller, in response to detecting a following stop signal, stops detection and obtains a current pose of an actively following region, and the operator may manually adjust the pose of the end tool, and such adjustment may be arbitrary.

In some embodiments, such adjustment process described above may be used in a hip replacement surgery. In some embodiments, the end tool may include an acetabular file or an acetabular cup, and the surgical robot 100 may provide a function of active following for the operator when the operator files a joint or prepares to place a cup for an object receives the surgery. In some embodiments, when the end tool is located in the second predetermined region 400, the surgical robot 100 may prompt the operator whether to turn on the function of active following, and when the operator selects to turn on the function of active following, the surgical robot 100 may control the end tool to follow a change in pose of the object to adjust the pose of itself (i.e., the end tool), so that the relative position between the end tool and the object remains unchanged. In some embodiments, the operator may also choose not to turn on the function of active following, where the surgical robot 100 does not control the end tool to follow the change in pose of the object.

In some embodiments, the end tool may be the acetabular file or the acetabular cup, and an initial pose corresponding relationship between the end tool and the object (i.e., the second pose corresponding relationship) may be such that a center axis of the end tool coincides with a center axis of the active following region.

The following illustrates a process of using the function of active following described above in relation to different stages of the hip replacement surgery.

In some embodiments, the acetabular file (the end tool) may be used to perform a bone-grinding operation on an acetabular socket prior to a cup hipping operation of the hip replacement surgery, and prior to the bone-grinding operation on the acetabular socket using the acetabular file, the acetabular file may be dragged to the actively following region, and a pose of the acetabular file may be adjusted so that a pose corresponding relationship between the acetabular file and the acetabular socket is a desired pose corresponding relationship, and then preparations for the bone-grinding operation on the acetabular socket may be made, e.g., preparing surgical instruments. In order to prevent the pose corresponding relationship between the acetabular file and the acetabular socket from changing due to a change in a patient's hip position during such preparation, a user may input a recording signal, and the controller or the processor obtains poses of the acetabular file and the acetabular socket based on the recording signal in response to detecting the recording signal, and uses the pose of the acetabular file as an initial tool pose (i.e., a first initial pose), uses the pose of the acetabular socket as an initial object pose (i.e., a second initial pose), and uses a corresponding relationship between the initial tool pose and the initial object pose as an initial pose corresponding relationship (i.e., the second pose corresponding relationship). In some embodiments, the user may input the recording signal by stepping on a foot pedal of a robot to cause the controller to obtain the poses of the acetabular file and the acetabular socket based on the recording signal. The pose following signal is then input via a second stepping mode of the foot pedal, and the controller uses current poses of the acetabular file and the acetabular socket as the initial tool pose and the initial object pose, respectively based on the pose following signal, and sets a position corresponding relationship between the initial tool pose and the initial object pose as the initial pose corresponding relationship, so to determine an initial pose offset of the initial tool pose with respect to the initial object pose. When a change in a pose of a hip joint is detected relative to the initial object pose, an object pose offset of the current pose of the hip joint relative to the initial object pose may be obtained, and a pose adjustment amount of the end tool may be determined based on the initial pose offset and the object pose offset, and the end tool may be controlled to move based on the pose adjustment amount so that when the movement is completed, a prompt signal indicating that the pose following is completed is outputted on a display device, and a pose corresponding relationship between the end tool and the hip joint is restored to the initial pose corresponding relationship.

In some embodiments, prior to the cup placing operation of the hip replacement surgery, the acetabular cup (end tool) may be moved to a predetermined cup placing location in the active following region. When the acetabular cup is located at the predetermined cup placing location, the center axis of the acetabular cup coincides with the center axis of the active following region, and the center axis of the acetabular cup may also already coincide with the center axis of the active following region before the acetabular cup reaches the predetermined cup placing location. The controller detects in real time the pose corresponding relationship between the acetabular cup and the acetabular socket during movement of the acetabular cup to the predetermined cup placing location, generates the recording signal when the center axis of the acetabular cup is detected to coincide with the center axis of the acetabular socket, and automatically records a pose of the actively following region and a pose of the end tool according to the recording signal. And in response to detecting the pose following signal input by a physician via the foot pedal, the controller uses a newly recorded pose of the active following region and a pose of the end tool as the initial object pose and the initial tool pose, respectively, and takes a corresponding relationship between the initial object pose and the initial tool pose as the initial position corresponding relationship. After that, the surgical robot 100 may automatically adjust the pose of the end tool, so as to enable the end tool to follow the change in the pose of the object. In some embodiments, when the active following is complete, an output device may output the prompt signal indicating that the pose following is complete. In some embodiments, the surgical robot 100 may also automatically perform a pose locking operation in response to completion of the pose following. In some embodiments, the pose following of the acetabular cup may occur during the cup placing operation performed by the operator, wherein the operator may perform the cup placing operation on the acetabular cup by striking an acetabular cup striking handle, then the acetabular cup collides with the acetabular socket after being struck. This may result in a change in the pose of the acetabular socket, at which time the operator may turn on the function of active following of the surgical robot 100. In some embodiments, after the active following is completed, the operator may perform the cup placing operation again based on the prompt signal.

The following continues with an example of the hip replacement surgery to illustrate another process of adjusting the pose of the end tool (in particular, a process of adjusting the pose of the acetabular cup during the cup placing operation). In some embodiments, the end tool may include the acetabular cup, and the object may include a patient's acetabular socket. In some embodiments, the acetabular socket may be a state in which an acetabular socket grinding surgery has been completed. In the hip replacement surgery, the center axis of the acetabular cup and the center axis of the acetabular socket need to coincide or substantially coincide with each other. However, in surgery, the operator generally controls the movement of the acetabular cup to a vicinity of the acetabular socket manually, and it is difficult to ensure that the center axis of the acetabular cup and the center axis of the acetabular socket is coincident or substantially coincident, and so the pose of the acetabular cup needs to be adjusted so that the center axis of the acetabular cup coincides or substantially coincides with the center axis of the acetabular socket.

In some embodiments, the positioning device 120 may detect the pose of the acetabular cup, and the processor 130 may determine whether the pose of the acetabular cup satisfies a first predetermined condition. In response to a determination that the pose of the acetabular cup satisfies the first predetermined condition, the processor 130 may calibrate the acetabular cup.

In some embodiments, the first predetermined condition may include two judgment threshold values including a distance threshold value between the center axis of the acetabular cup and the center axis of the acetabular socket, and an angle threshold value between the center axis of the acetabular cup and the center axis of the acetabular socket. The first predetermined condition may include two judgment conditions including whether a distance between the central axis of the acetabular cup and the central axis of the acetabular socket is less than the distance threshold, and whether an angle between the central axis of the acetabular cup and the central axis of the acetabular socket is less than the angle threshold. When a result of each of the two judgment conditions is "yes", it may be determined that the pose of the acetabular cup satisfies the first predetermined condition, and the acetabular cup may be calibrated. In some embodiments, the distance threshold value and the angle threshold value may be set according to an actual situation. The distance threshold value and angle threshold value may be set to ensure that the acetabular cup is located near the acetabular socket. In response to a determination that the distance between the center axis of the acetabular cup and the center axis of the acetabular socket is greater than the distance threshold value or the angle between the center axis of the acetabular cup and the center axis of the acetabular socket is greater than the angle threshold value, the operator may manually control again and adjust the acetabular cup to move to the vicinity of the acetabular socket. In some embodiments, the first predetermined condition may further include that the center axis of the acetabular cup does not coincide, or does not substantially coincide, with the center axis of the acetabular socket. In some embodiments, if the operator has directly caused the center axis of the acetabular cup to coincide or substantially coincide with the center axis of the acetabular socket by manually controlling the movement of the acetabular cup to the vicinity of the acetabular socket, there is no need for the processor 130 to calibrate the acetabular cup.

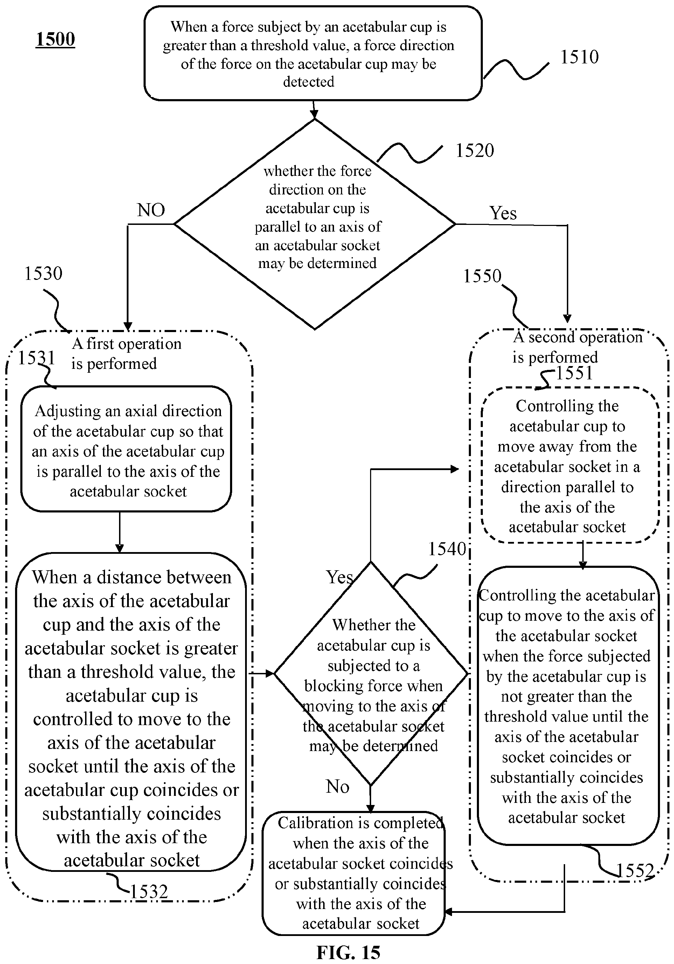

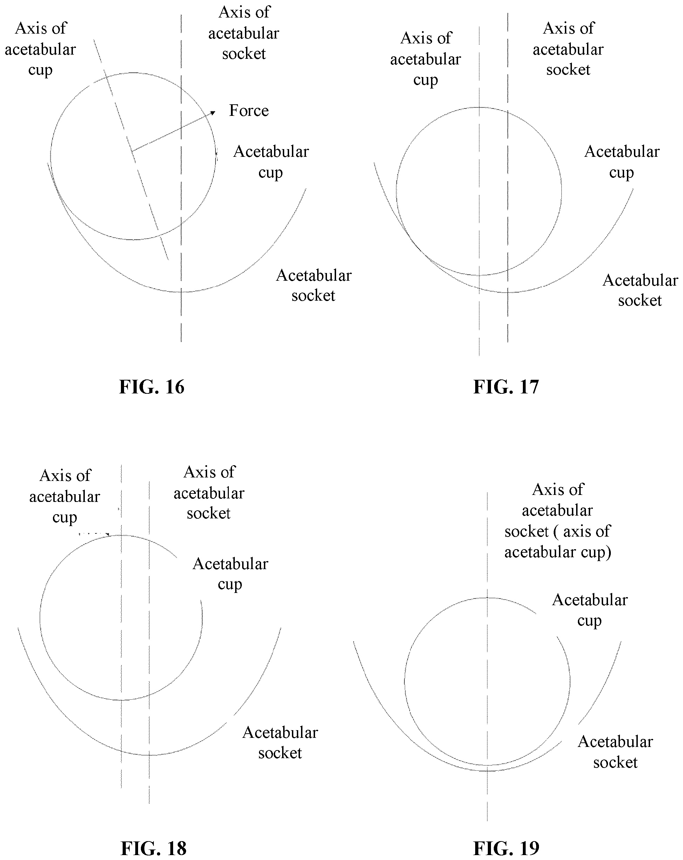

FIG. 15 is a flowchart illustrating an exemplary process for calibrating an acetabular cup according to some embodiments of the present disclosure, and FIGs. 16 to 19 are schematic diagrams illustrating different poses of an acetabular cup according to some embodiments of the present disclosure. As shown in FIG. 15 and FIGs. 16 to 19, a process 1500 may include following operations. In some embodiments, the process 1500 may be performed by the surgical robot 100. Operational intent of the process 1500 presented below is illustrative. In some embodiments, the process may be accomplished utilizing one or more additional operations not described and/or one or more operations not discussed.

In 1510, when a force subject by the acetabular cup is greater than a threshold value, a force direction of the force on the acetabular cup may be detected. In some embodiments, operation 1510 may be performed by the processor 130.

In some embodiments, after the acetabular cup enters an acetabular socket, the acetabular cup is subjected to a collision force when the acetabular cup touches an inner wall of the acetabular socket (shown in FIG. 16) during movement. In some embodiments, the collision force may be measured by a force sensor provided on the robotic arm device 110.

When the force subjected by the acetabular cup is greater than the threshold value, it indicates that the acetabular cup has contacted with the inner wall of the acetabular socket and the acetabular cup may not further move. At this time, the force direction on the acetabular cup is detected and the force direction on the acetabular cup is compared with an axial direction of the acetabular socket, thereby determining a pose of the acetabular cup inside the acetabular socket, which then facilitates adjusting the pose of the acetabular cup.

In 1520, whether the force direction on the acetabular cup is parallel to an axis of the acetabular socket may be determined. In some embodiments, operation 1520 may be performed by the processor 130.

It should be noted that "parallel" in some embodiments of the present disclosure does not have to be exactly parallel, but rather may include being parallel or substantially parallel. In some embodiments, substantially parallel may mean that an angle between two directions that are substantially parallel is within 10 degrees. In some embodiments, substantially parallel may mean that the angle between the two directions that are substantially parallel is within 5 degrees. In some embodiments, when the force direction on the acetabular cup is parallel or substantially parallel to the axis of the acetabular socket (e.g., an angle between the force direction on the acetabular cup and the axis of the acetabular socket is within 10 degrees), the processor 130 may judge a result to be yes, and when the force direction on the acetabular cup is not parallel or not substantially parallel to the axis of the acetabular socket, the process 130 may judge the result to be no. In response to a determination that a judgement result of operation 1520 is no, it indicates that the force direction on the acetabular cup is not parallel to the axis of the acetabular socket, operation 1530 is performed. In response to a determination that the judgment result of operation 1520 is yes, it indicates that the force direction on the acetabular cup is parallel to the axis of the acetabular socket, then operation 1550 is performed.

In 1530, a first operation is performed. In some embodiments, operation 1530 may be performed by the processor 130.

In some embodiments, the first operation may be used to adjust an axis of the acetabular cup to coincide or substantially coincide with the axis of the acetabular socket.

In some embodiments, the first operation may include sub-operation 1531, adjusting an axial direction of the acetabular cup so that the axis of the acetabular cup is parallel to the axis of the acetabular socket; sub-operation 1532, when a distance between the axis of the acetabular cup and the axis of the acetabular socket is greater than a threshold value, the acetabular cup is controlled to move to the axis of the acetabular socket until the axis of the acetabular cup coincides or substantially coincides with the axis of the acetabular socket.

In some embodiments, the processor 130 may control the robotic arm device 110 to drive the acetabular cup to rotate, so as to adjust the axial direction of the acetabular cup until a pose of the acetabular cup measured by the positioning device 130 indicates that the axis of the acetabular cup is parallel to the axis of the acetabular socket, as shown in FIG. 16 and FIG. 17. After the axis of the acetabular cup is parallel to the axis of the acetabular socket, whether the distance between the axis of the acetabular cup and the axis of the acetabular socket is greater than the threshold value may be determined. In response to a determination that the distance between the axis of the acetabular cup and the axis of the acetabular socket is less than or equal to the threshold value, it indicates that the distance between the axis of the acetabular cup and the axis of the acetabular socket satisfies a condition of substantial coincidence, and the axis of the acetabular cup substantially coincides with the axis of the acetabular socket. In response to a determination that the distance between the two is greater than the threshold value, it means that the axis of the acetabular cup is far away from the axis of the acetabular socket, and the acetabular cup may be controlled to move to the axis of the acetabular socket until the axis of the acetabular cup coincides or substantially coincides with the axis of the acetabular socket as shown in FIG. 17 and FIG. 19. During the movement of the acetabular cup to the axis of the acetabular socket, operation 1540 may be performed.

Operation 1540, whether the acetabular cup is subjected to a blocking force when moving to the axis of the acetabular socket may be determined. In some embodiments, operation 1540 may be performed by the processor 130.

In some embodiments, when the acetabular cup is subjected to the blocking force when moving to the axis of the acetabular socket, it is indicated that the acetabular cup collides with the inner wall of the acetabular socket, and the acetabular cup penetrates too deeply into the acetabular socket, and operation 1550 may be performed. When the acetabular cup is not subjected to the blocking force when moving to the axis of the acetabular socket, the acetabular cup may be controlled to continue to move to the axis of the acetabular socket until the axis of the acetabular cup coincides or substantially coincides with the axis of the acetabular socket, then adjusting the pose of the acetabular cup is completed.

In 1550 a second operation may be performed. In some embodiments, operation 1550 may be performed by the processor 130.

In some embodiments, when the acetabular cup moves to the axis of the acetabular socket, the second operation may be used to adjust the axis of the acetabular cup to coincide or substantially coincide with the axis of the acetabular socket.

In some embodiments, the second operation may include sub-operation 1551, controlling the acetabular cup to move away from the acetabular socket in a direction parallel to the axis of the acetabular socket; and sub-operation 1552, controlling the acetabular cup to move to the axis of the acetabular socket when the force subjected by the acetabular cup is not greater than the threshold value until the axis of the acetabular socket coincides or substantially coincides with the axis of the acetabular socket.

In some embodiments, when the acetabular cup is too deep into the acetabular socket and is subjected to a large force, the acetabular cup may first be controlled to move back away from the acetabular socket in the direction parallel to the axis of the acetabular socket, henceforth reducing a pressure of the acetabular socket on the acetabular cup, as shown in FIG. 17 and FIG. 18. When the force subjected by the acetabular cup is not greater than the threshold value, the acetabular cup is controlled to move to the axis of the acetabular socket until the axis of the acetabular cup coincides or substantially coincides with the axis of the acetabular socket, as shown in FIGS. 18 with FIG. 19, then the calibration is completed.

In response to a determination that the force direction on the acetabular cup is parallel to the axis of the acetabular socket, when the force subjected by the acetabular cup is not greater than the threshold value, the processor 130 may control the robotic arm device 110 to drive the acetabular cup to move to the axis of the acetabular socket until the axis of the acetabular cup coincides or substantially coincides with the axis of the acetabular socket, then the calibration is completed.

FIG. 20 is a schematic diagram illustrating an exemplary structure of a surgical robot during a joint grinding and filing phase according to some embodiments of the present disclosure. As shown in FIG. 20, the present disclosure also provides a surgical robot 2000. In some embodiments, the surgical robot 2000 includes a robotic arm device 2010, a positioning device, and a processor.

In some embodiments, the robotic arm device 2010 may be used to control an end tool to move. In some embodiments, the robotic arm device 2010 may also output set feedback information to the end tool. In some embodiments, the robotic arm device 2010 may include a base 2011 and an arm assembly 2012. The arm assembly 2012 is fixed to the base 2011 and the base 2011 provides a support mounting platform. In some embodiments, an end of the arm assembly 2012 that is away from the base 2011 is provided with an end tool 2040. In some embodiments, the arm assembly 2012 may be coupled to the end tool 2040 via a linkage 2050, and the arm assembly 2012 drives the end tool 2040 to move via the linkage 2050. In some embodiments, the center axis of the linkage 2050 may coincide with the center axis of the end tool 2040.

In some embodiments, an end of the linkage 2050 away from the end tool 2040 also includes an operation auxiliary piece 2051. In some embodiments, see FIG. 20, when a surgical operation is in the joint grinding and filing phase, the end tool 2040 may be an acetabular file, the linkage 2050 may be an acetabular file linkage, and the operation auxiliary piece 2051 may be a power device of the acetabular file. In some embodiments, when the surgical operation is in a cup placing phase (not shown in FIG. 20), the end tool 2040 may be an acetabular cup, the linkage 2050 may be an acetabular cup linkage, and the operation auxiliary piece 2051 may be an end cap of the acetabular cup linkage, and during a cup placing process, an operator may transmit a power to the acetabular cup by tapping the end cap to perform a cup placing operation.

The positioning device may be used to obtain pose information of a predetermined object. In some embodiments, the predetermined object may include the end tool, a surgical object, or other markers, among others. In some embodiments, the positioning device may be used to obtain first pose information of the end tool 2040 and second pose information of an object.

In some embodiments, the positioning device may include a positioning information obtaining structure 2025 and one or more markers. The positioning information obtaining structure may interoperate with the one or more markers to obtain corresponding pose information. In some embodiments, the one or more markers may include a first marker 2021 disposed on the base 2011 and a third marker 2023 disposed on the object. In some embodiments, the positioning device may further include a second marker 2022 indicating a pose of the end tool 2040. In some embodiments, the second marker 2022 may be provided on the end tool 2040. In other embodiments, the second marker 2022 may also be provided on a robotic arm joint that is used to fix the end tool 2040. The first marker 2021 may be used as a reference datum for a reference coordinate system, the second marker 2022 may be used to determine pose information of the end tool, and the third marker 2023 may be used to determine pose information of the object.

In some embodiments, a marker may include at least one of an optical array, an electronic positioning device, an electromagnetic positioning device, a GPS positioning device, a gyroscope, or an accelerometer. In some embodiments, when each of the one or more markers includes the optical array, the first marker 2021 may be a first optical array, the second marker 2022 may be a second optical array, and the third marker 2033 may be a third optical array and the positioning information obtaining structure 2025 may accordingly include an optical camera.

In some embodiments, one or more processors may obtain pose information of the end tool 2040 via the positioning device and control the robotic arm device 2010 to provide first feedback information to the operator based on the pose information. In some embodiments, the one or more processors may control the movement of the robotic arm device 2010, thereby controlling poses of the robotic arm device 2010 and the end tool. In some embodiments, a count of the one or more processors may be one or more.

In some embodiments, the surgical robot 2000 may further include a memory, an input device, and an output device 2060. In some embodiments, the one or more processors the memory, the input device, and the output device may be connected via a bus or otherwise.

The memory, as a computer-readable storage medium, may be used to store a software program, a computer-executable program, and one or more modules such as program instructions/modules corresponding to the process 200 in the present disclosure (e.g., a distance-stiffness function determination module and a first feedback force output module). The processor performs various functional applications of the device as well as data processing by running the software program, instructions, and modules stored in the memory.

In some embodiments, the memory may primarily include a storage program area and a storage data area. The storage program area may store an operation system and an application required for at least one function; and the storage data area may store data created based on use of the terminal, etc. In some embodiments, the memory may include high-speed random access memory, or non-volatile memory, such as at least one disk memory device, a flash memory device, or another non-volatile solid-state memory device, etc. In some embodiments, the memory may also include memory set remotely with respect to the processor, and the remote memory may be connected to the device over a network. Exemplary networks include, but are not limited to, the Internet, an enterprise intranet, a local area network (LAN), a cellular network, and combinations thereof.

The input device may be used to receive incoming numeric or character information, as well as to generate inputs of key signals related to user settings as well as function control of the device.

The output device 2060 may be used to output various warning information, such as warning information when the end tool 2040 fails to follow a predetermined path to detach from the first predetermined region 300. In some embodiments, the output device 2060 may be used to output a pose corresponding relationship between the end tool 2040 and the second predetermined region 400 and/or a pose corresponding relationship between the end tool 2040 and the first predetermined region 300. In some embodiments, the output device 2060 may also be used to output individual pose information determined by the positioning device, such as the pose information of the end tool 2040, etc. In some embodiments, the output device 2060 may also be used to output a signal indicating that the end tool 2040 has completed the pose adjustment. In some embodiments, the output device 2060 may include a display, and various warning information output by the output device 2060 may be displayed via the display.

In some embodiments, the output device 2060 may also output the first predetermined region 300 and/or the second predetermined region 400 (or a constraint space and a sub-constraint space), and the operator may observe from the display of the output device 2060 and confirm a specific location of the end tool in the first predetermined region 300 and/or the second predetermined region 400 (or in the constraint space and the sub-constraint space).

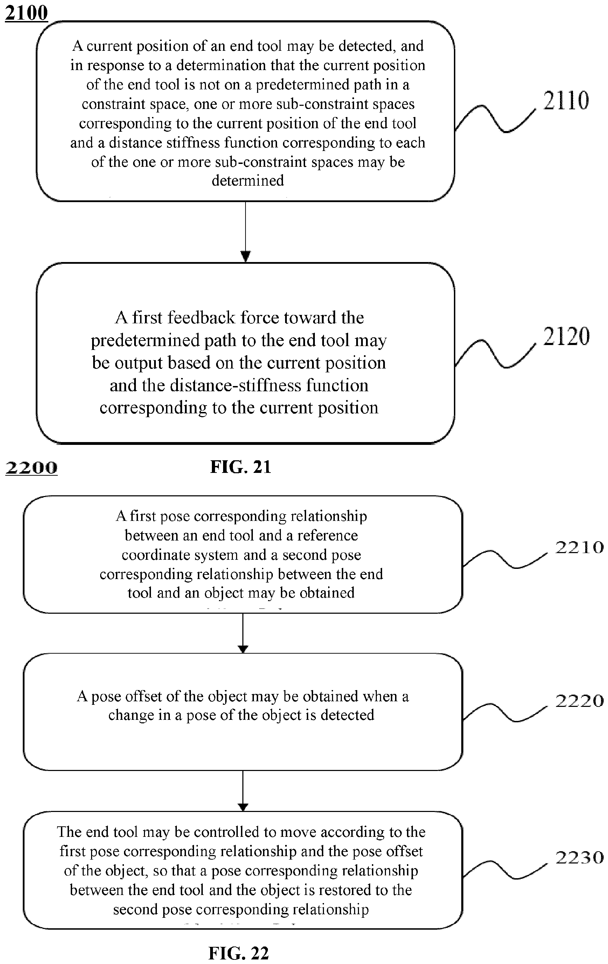

FIG. 21 is a flowchart illustrating an exemplary process for guiding the movement of an end tool according to some embodiments of the present disclosure. As shown in FIG. 21, the present disclosure also provides a process 2100 of guiding the movement of the end tool. This process guides a position and movement direction of the end tool, which optimizes a movement path of the end tool, thereby improving the speed, safety, and accuracy of an orthopedic surgery. The present disclosure also provides a surgical robot applied in the above process. In some embodiments, the above-described process may be performed by the surgical robot.

In some embodiments, the surgical robot may include a robotic arm device, a positioning device, and a processor. In some embodiments, the robotic arm device may be used to drive an end tool to move under an external force. In some embodiments, the robotic arm device may output a feedback force to the end tool. In some embodiments, the positioning device may be used to obtain a current position of the end tool in a constraint space, wherein a central axis of the constraint space is a predetermined path of the end tool, and the constraint space may include at least two sub-constraint spaces nested together. In some embodiments, the processor may be used to detect the current position of the end tool by the positioning device. In some embodiments, in response to a determination that the current position of the end tool is not on the predetermined path in the constraint space, the processor may determine a sub-constraint space corresponding to the current position of the end tool and a distance-stiffness function corresponding to the sub-constraint space. In some embodiments, the processor may output, via the robotic arm device, a first feedback force toward the predetermined path to the end tool based on the current position and the distance-stiffness function corresponding to the current position.

In some embodiments, the robotic arm device may be similar to or same as the robotic arm device 2010 of the surgical robot 2000 shown in FIG. 20, the positioning device may be similar to or same as the positioning device of the surgical robot 2000 shown in FIG. 20, and the processor may be similar to or same as the processor of the surgical robot 2000 shown in FIG. 20 and will not be described herein.

In some embodiments, the process 2100 includes following operations.

In 2110, the current position of the end tool may be detected, and in response to a determination that the current position of the end tool is not on the predetermined path in the constraint space, one or more sub-constraint spaces corresponding to the current position of the end tool and a distance-stiffness function corresponding to each of the one or more sub-constraint spaces may be determined.

In some embodiments, the constraint space may include two or more sub-constraint spaces nested in the same direction, and a center axis of each sub-constraint space coincides. Determining a sub-constraint space includes dividing the constraint space into two or more layers from the inside to the outside based on a predetermined ratio of effective radius ranges of sub-constraint spaces, and designating each layer as a sub-constraint space. In some embodiments, the constraint space may be divided into three layers, i.e., the constraint space is divided into three sub-constraint spaces. The three sub-constraint spaces are, from the inside to the outside, a free constraint space, a buffer constraint space, and a boundary constraint space. In some embodiments, different distance-stiffness functions may be configured for the different sub-constraint spaces, wherein the distance-stiffness function for a sub-constraint space is a constant function or an increasing function. A sub-constraint space whose distance-stiffness function is a constant function is alternated with a sub-constraint space whose distance-stiffness function is an increasing function. In some embodiments, distance-stiffness functions corresponding to neighboring sub-constraint spaces have the same distance stiffness value at a common boundary. In some embodiments, a distance-stiffness function corresponding to the free constraint space is a first constant stiffness function; and a distance-stiffness function corresponding to the buffer constraint space located in the intermediate layer of the constraint space is a monotonically increasing quadratic curve; and a distance-stiffness function corresponding to the boundary constraint space located in the outermost layer of the constraint space is a second constant stiffness function. A structure of the constraint space can be referred to a structure of the first predetermined region 300 shown in FIG. 3 and a structure of the second predetermined region 400 shown in FIG. 4, and will not be repeated here.

In 2120, the first feedback force toward the predetermined path to the end tool may be outputted based on the current position and the distance-stiffness function corresponding to the current position.

In some embodiments, after determining the current position of the end tool and the distance-stiffness function corresponding to the current position, a distance stiffness value corresponding to the current position is determined, and a product of a target distance corresponding to the current position and the distance stiffness value is used as an absolute value of the first feedback force, and the first feedback force is output toward the predetermined path to the end tool. Preferably, a direction of the first feedback force is perpendicular and toward the predetermined path.

In one embodiment, after determining the current position of the end tool and the distance-stiffness function corresponding to the current position, the target distance between the current position and the predetermined path is determined and a target distance equivalent corresponding to the target distance is determined; based on the target distance equivalent and the distance-stiffness function corresponding to the current position, the distance stiffness value corresponding to the current position is determined; based on the target distance equivalent and the distance stiffness value, the absolute value of the first feedback force is determined, and the first feedback force toward the predetermined path is output to the end tool.

In some embodiments, operations of the process 2100 may be described with reference to the process 200 and the related descriptions of FIGs. 2 to 11, and will not be repeated herein.

Some embodiments of the present disclosure further provide a movement guiding device for the end tool, and the device is used to perform a process to guide the movement of the end tool provided in any of the above embodiments. The movement guiding device is implemented on optionally a software or hardware. In some embodiments, the device may include a distance-stiffness function determination module and a first feedback force output module.

In some embodiments, the distance-stiffness function determination module may be used to detect the current position of an end tool, and in response to a determination that the current position of the end tool is not on the predetermined path in the constraint space, one or more sub-constraint spaces corresponding to the current position of the end tool and the distance-stiffness function corresponding to each of the one or more sub-constraint spaces are determined, wherein the center axis of the constraint space is the desired predetermined path of the end tool, and the constraint space includes at least two sub-constraint spaces nested in the same direction.

In some embodiments, the first feedback force output module may be used to output the first feedback force toward the desired path to the end tool based on the current position and the distance-stiffness function corresponding to the current position. In some embodiments, the first feedback force output module may further be used to determine the target distance between the current position and the predetermined path, and the target distance equivalent corresponding to the target distance (i.e., a current distance equivalent); determine the distance stiffness value corresponding to the current position based on the target distance equivalent and the distance-stiffness function corresponding to the current position; and output the first feedback force toward the predetermined path to the end tool based on the target distance equivalent and the distance stiffness value.

In some embodiments, the movement guiding device may further comprise an angle-stiffness function determination module configured to determine, in response to detecting that a current angle between the end tool and the predetermined path is greater than zero, an angle-stiffness function corresponding to one of one or more angle ranges in which the current angle is located, wherein a count of the one or more angle ranges is greater than or equal to 2. In some embodiments, a count of the one or more angle ranges is 3; angle-stiffness functions corresponding to angle ranges comprising a largest angel and a smallest angle are both constant functions, and an angle-stiffness function corresponding to an angle range located between the angle ranges comprising a largest angle and a smallest angle is an increasing function; and angle-stiffness functions corresponding to neighboring angle ranges have the same stiffness value at a common boundary.

In some embodiments, the movement guiding device for the end tool may further include a rotational torque output module configured to output, based on the current angle and the angle-stiffness function corresponding to the current angle, a rotational torque (e.g., a first feedback torque) to the end tool for rotating the end tool toward the predetermined path.

In some embodiments, the movement guiding device for the end tool may further include a damping function determination module configured to determine, in response to detecting that a direction of a current movement speed of the end tool deviates from the predetermined path, a speed range corresponding to the current movement speed and a damping function corresponding to the speed range. In some embodiments, damping functions corresponding to a speed range comprising a maximum allowable speed and a speed range comprising a minimum allowable speed are both constant functions, a damping function corresponding to a speed range located between the speed range comprising a maximum allowable speed and the speed range comprising a minimum allowable speed is an increasing function; damping functions corresponding to adjacent speed ranges have the same damping value at a common boundary.

In some embodiments, the movement guiding device for the end tool may further include a second feedback force outputting module configured to output, based on the current movement speed and the damping function corresponding to the current movement speed, the second feedback force towards the predetermined path to the end tool.

In some embodiments, the movement guiding device for the end tool may further include a warning module configured to perform a braking operation on the end tool in response to detecting that a feature point of the end tool is located at an outer boundary of the constraint space, and output first warning information. A target position of the end tool is a top of the buffer constraint space.

A technical solution of the movement guiding device for the end tool provided by some embodiments of the present disclosure detects the current position of the end tool by the stiffness distance function determination module, and, in response to a determination that the end tool is not currently on the predetermined path in the constraint space, determines the sub-constraint space corresponding to the current position of the end tool and the distance-stiffness function corresponding to the sub-constraint space; output the first feedback force toward the predetermined path to the end tool by the first feedback force outputting module in accordance with the current position and the distance-stiffness function corresponding to the current position; since the constraint space includes at least two sub-constraint spaces nested together, different sub-constraint spaces correspond to different distance-stiffness functions, thus the first feedback force received by the end tool in different sub-constraint spaces varies in different forms. Therefore, a user may determine a sub-constraint space in which the end tool is currently located according to a variation form of the first feedback force, i.e., an approximate position of the end tool in the constraint space, thereby realizing the position guidance for the end tool; and also enabling the user to determine the movement direction of the end tool according to a direction of the first feedback force, thereby realizing the movement direction guidance for the end tool. Through the position and movement direction guidance for the end tool optimizing the movement path of the end tool, the speed, safety, and accuracy of the orthopedic surgery can be improved.

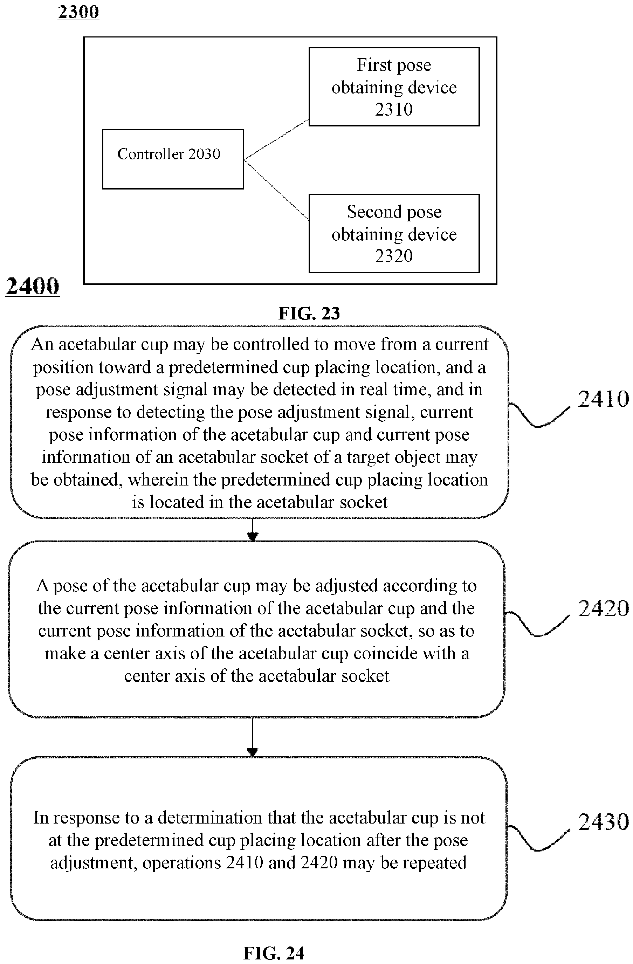

FIG. 22 is a flowchart illustrating an exemplary process for correcting a pose of an end tool in real-time according to some embodiments of the present disclosure, and FIG. 23 is a schematic diagram illustrating other exemplary modules of a surgical robot according to some embodiments of the present disclosure. As shown in FIG. 22 and FIG. 23, the present disclosure also provides a process 2200 for correcting a pose of an end tool in real time and provides a surgical robot 2300 as shown in FIG. 23. This process helps to improve the efficiency and accuracy of pose adjustment of the end tool, thereby improving the efficiency and success of a joint replacement surgery. In some embodiments, the process may control the surgical robot 2300 to correct the pose of the end tool in real time in response to a change in a pose of a hip join, so to restore a pose corresponding relationship between the end tool and an acetabular socket to an initial second pose corresponding relationship. In some embodiments, the process 2200 may be performed by a controller 2330 of the surgical robot 2300. In some embodiments, the controller 2330 may be a processor, a server, or other computing device set up inside or outside the surgical robot 2300. In some embodiments, the controller 2330 may be implemented in software and/or hardware.

In some embodiments, the surgical robot 2300 mainly includes a first pose obtaining device 2310, a second pose obtaining device 2320, and a controller 2330. In some embodiments, the first pose obtaining device 2310 may be disposed on an object and maintained in a fixed corresponding relationship with the object for obtaining a pose of the object. In some embodiments, the second pose obtaining device 2320 may be disposed on a robotic arm carrying an end tool or the end tool for obtaining the pose of the end tool. In some embodiments, the controller 2330 may be used to obtain a first pose corresponding relationship between the end tool and a reference coordinate system. In some embodiments, the controller 2330 may also be used to obtain a second pose corresponding relationship between the end tool and the object. In some embodiments, the controller 2330 may also be used to obtain a pose offset of the object when a change in the pose of the object is detected. In some embodiments, the controller 2330 may also be used to control the end tool to move based on the first pose corresponding relationship and the pose offset of the object, so that the pose corresponding relationship between the end tool and the object is restored to the second pose corresponding relationship.

In some embodiments, the first pose obtaining device 2310 and the second pose obtaining device 2320 may be referenced to the positioning device of the surgical robot 2000 shown in FIG. 20, and the controller 2330 may be referenced to the processor of the surgical robot 2000, which will not be described herein.

In some embodiments, the process 2200 includes following operations.

In 2210, the first pose corresponding relationship between the end tool and the reference coordinate system and the second pose corresponding relationship between the end tool and the object may be obtained.

In 2220, the pose offset of the object may be obtained when a change in the pose of the object is detected.

In 2230, the end tool may be controlled to move according to the first pose corresponding relationship and the pose offset of the object, so that the pose corresponding relationship between the end tool and the object is restored to the second pose corresponding relationship.

In some embodiments, various operations of the process 2200 can be described with reference to the process 1200 and the related descriptions of FIGs. 12 to 14, and will not be repeated herein.

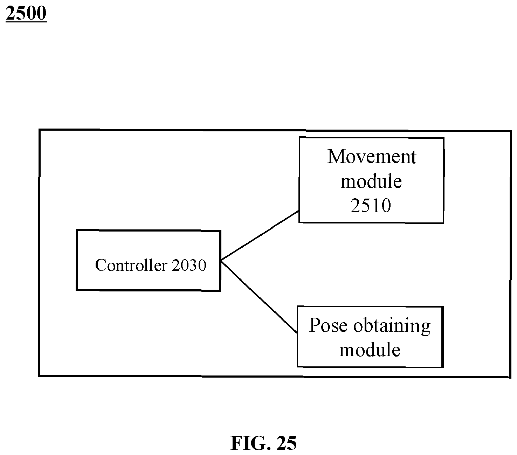

FIG. 24 is a flowchart illustrating an exemplary process for automatically adjusting a pose of an acetabular cup according to some embodiments of the present disclosure shown in FIG. 25, and FIG. 25 is a schematic diagram illustrating another exemplary surgical robot according to some embodiments of the present disclosure. As shown in FIG. 24 and FIG. 25, the present disclosure also provides a process 2400for automatically adjusting a pose of an acetabular cup and a surgical robot 2500 as shown in FIG. 25. This process significantly reduces a time for adjusting the pose of the acetabular cup and is able to adjust the acetabular cup to an optimal cup placing position, which helps to improve the success rate of an acetabular cup installation operation. In some embodiments, the manner adjusts a pose of the acetabular cup by current pose information of the acetabular cup with current pose information of an acetabular socket so that when the acetabular cup is located at a predetermined cup placing location, a center axis of the acetabular cup and a center axis of the acetabular socket coincides. In some embodiments, process 2400 may be performed by a controller 2530 of the surgical robot 2500. In some embodiments, the controller 2530 may be a processor, server, or other computing device set up inside or outside the surgical robot 2300. In some embodiments, the controller 2530 may be implemented in software and/or hardware.

In some embodiments, the surgical robot 2500 primarily includes a movement module 2510, a pose obtaining module 2520, and the controller 2530. In some embodiments, the movement module 2510 may include a robotic arm for driving the acetabular cup to move. In some embodiments, the pose obtaining module 2520 may include an acetabular cup pose unit and an acetabular socket pose unit. In some embodiments, the pose obtaining module 2520 may obtain current pose information of the acetabular cup in real time via the acetabular cup pose unit, and in some embodiments, the pose obtaining module 2520 may obtain current pose information of the acetabular socket in real time via the acetabular socket pose unit. In some embodiments, the controller 2530 may be used to adjust the pose of the acetabular cup based on the current pose information of the acetabular cup and the current pose information of the acetabular socket to cause the center axis of the acetabular cup to coincide with the center axis of the acetabular socket.

In some embodiments, the movement module 2510 may be similar to as the same as the robotic arm device 2010 of the surgical robot 2000 shown in FIG. 20, the pose obtaining module 2520 may be similar to as the same as the positioning device of the surgical robot 2000 shown in FIG. 20, the controller 2530 may be similar to as the same as the processor of the surgical robot 2000 shown in FIG. 20, and will not be described herein.

In some embodiments, the process 2400 primarily includes following operations.

In 2410,the acetabular cup may be controlled to move from a current position toward a predetermined cup placing location, and a pose adjustment signal may be detected in real time, and in response to detecting the pose adjustment signal, the current pose information of the acetabular cup and the current pose information of the acetabular socket of a target object may be obtained, wherein the predetermined cup placing location is located in the acetabular socket.

In some embodiments, the target object (also referred to as an object) may be a patient currently receiving an acetabular cup installation operation. In some embodiments, the target object may be a patient who has been performed an acetabular socket grinding operation and is awaiting an acetabular cup placement. In some embodiments, the predetermined cup placing location is a cup placing location determined by a doctor before a surgery, a center of the predetermined cup placing location is near a center of the acetabular socket.

In some embodiments, the pose adjustment signal may be triggered by an external force, which is a collision force applied to the acetabular cup as the acetabular cup touches an inner wall of the acetabular socket when the acetabular cup moves toward the predetermined cup placing location. In some embodiments, in response to a force sensor disposed on the robotic arm detecting that the acetabular cup has been subjected to the external force due to touching the inner wall of the acetabular socket, the pose adjustment signal is generated, and based on the pose adjustment signal, the current pose information of the acetabular cup and the current pose information of the acetabular socket of the target object are obtained according to the pose adjustment signal. By a manner in which movement occurs first and then the pose adjustment signal is generated based on the external force, the longitudinal adjustment of the acetabular cup is realized, and then a transversal overlap adjustment of the acetabular cup is realized by means of the pose adjustment signal and subsequent operations, which realizes automatic adjustment of the pose of the acetabular cup.

In some embodiments, before controlling the acetabular cup to move from the current position toward the predetermined cup placing location and detecting the pose adjustment signal in real time, the current pose information of the acetabular cup and the current pose information of the acetabular socket of the target object are obtained; and the pose of the acetabular cup is adjusted according to the current pose information of the acetabular cup and the current pose information of the acetabular socket, so as to make the center axis of the acetabular cup coincide with the center axis of the acetabular socket. Before the longitudinal adjustment of the acetabular cup, the transverse overlap adjustment of the acetabular cup is performed to make the central axis of the acetabular cup parallel to the central axis of the acetabular socket at the start of the first movement, helping to increase an amount of longitudinal movement during the first movement. In some embodiments, causing the center axis of the acetabular cup to coincide with the center axis of the acetabular socket may include adjusting the pose of the acetabular cup based on the current pose information of the acetabular cup and the current pose information of the acetabular socket to cause the acetabular cup's central axis is parallel to the central axis of the acetabular socket, henceforth updating the current pose information of the acetabular cup, adjusting a position of the center axis of the acetabular cup in a predetermined direction according to updated current pose information of the acetabular cup so as to cause the center axis of the acetabular cup to coincide with the center axis of the acetabular socket, wherein the predetermined direction is a direction perpendicular to the center axis of the acetabular socket.

In some embodiments, the pose adjustment signal is generated in response to detecting that the acetabular cup has completed a predetermined step length, wherein the predetermined step length is less than a minimum distance from the inner wall of the acetabular socket to the predetermined cup placing location. Specifically, a current distance between the acetabular cup and the predetermined cup placing location is set to be H, and H is equal to 8 predetermined step lengths. Pose adjustment is performed on the acetabular cup so that the center axis of the acetabular cup coincides with the center axis of the acetabular socket, and the acetabular cup whose pose has been adjusted is controlled to move toward the predetermined cup placing location at the predetermined step length, and, in response to detecting that each predetermined step length is completed, the pose adjustment signal is generated, and the current pose information of the acetabular cup and the current pose information of the acetabular socket of the target object are obtained based on the pose adjustment signal. The real-time pose adjustment of the acetabular cup is realized by first performing a transverse overlap adjustment of the acetabular cup, then performing the longitudinal position adjustment of the acetabular cup, and then performing a subsequent overlap adjustment of the acetabular cup based on the pose adjustment signal.

In some embodiments, a mode of automatically adjusting the pose of the acetabular cup is triggered by a pose following signal, and the pose following signal is generated by a physician by stepping on a foot pedal of a surgical robot. In some embodiments, when the physician drags the acetabular cup to a vicinity of the acetabular socket and the surgical robot is required to automatically perform the pose adjustment on the acetabular cup, the controller generates the pose following signal based on the action information of the foot pedal when the foot pedal of the surgical robot is stepped on. Correspondingly, when the physician needs to terminate the mode of automatically adjusting the pose of the acetabular cup, a pose following termination signal may also be generated by stepping on the foot pedal. A manner/ timing in which the foot pedal is stepped on for generating the pose following signal is different from a manner/time in which the foot pedal is stepped on for generating the pose following termination signal.

It should be noted that pose adjustment signals generated based on different triggering modes correspond to different automatic adjustment modes, and different automatic adjustment modes require different pose following signals to be triggered, which may be generated through different manners in which the foot pedal is stepped on.

In 2420, the pose of the acetabular cup may be adjusted according to the current pose information of the acetabular cup and the current pose information of the acetabular socket, so as to make the center axis of the acetabular cup coincide with the center axis of the acetabular socket.

In some embodiments, the current pose information of the acetabular cup is updated by causing the central axis of the acetabular cup to be parallel to the central axis of the acetabular socket, which is realized by adjusting the pose of the acetabular cup based on the current pose information of the acetabular cup and the current pose information of the acetabular socket; and according to updated pose information of the acetabular cup, the position of the acetabular cup in the predetermined direction is adjusted, so that the center axis of the acetabular cup coincides with the center axis of the acetabular socket, henceforth accomplishing the coinciding adjustment of the acetabular cup in a lateral direction. Wherein the predetermined direction is a direction perpendicular to the center axis of the acetabular socket.

In 2430, in response to a determination that the acetabular cup is not at the predetermined cup placing location after the pose adjustment, operations 2410 and 2420 may be repeated.

In some embodiments, various operations of the process 2400 can be described with reference to the process 1500 and the related descriptions of FIGs. 15 to 19, and will not be repeated here.

In some embodiments, the pose adjustment signal is triggered by an external force. In some embodiments, the robotic arm is controlled to drive the acetabular cup to move toward the predetermined cup placing location until the external force is detected as a result of the acetabular cup touching the inner wall of the acetabular socket. The pose adjustment signal is generated based on the external force, and the current pose information of the acetabular cup and the current pose information of the acetabular socket of the target object are obtained based on the pose adjustment signal, and the current pose of the acetabular socket is adjusted based on the current pose information of the acetabular cup and the current pose information of the acetabular socket, so to make the center axis of the acetabular cup coincide with the center axis of the acetabular socket. Then, whether the acetabular cup after the pose adjustment is currently located at the predetermined cup placing location is detected, in response to a determination that the acetabular cup is located at the predetermined cup placing location, the current pose of the acetabular cup may be locked; in response to a determination that the acetabular is not located at the predetermined cup placing location, the robotic arm may be controlled to drive the acetabular cup toward the predetermined cup placing location until an external force on the acetabular cup due to touching the inner wall of the acetabular socket is detected. The pose adjustment signal is generated based on the external force, and the current pose information of the acetabular cup and the current pose information of the acetabular socket of the target object are obtained based on the pose adjustment signal, and the pose of the acetabular cup is adjusted based on the current pose information of the acetabular cup and the current pose information of the acetabular socket so that the center axis of the acetabular cup coincides with the center axis of the acetabular socket. Then whether the acetabular cup after the pose adjustment is currently located at the predetermined cup placing location is detected; in response to a determination that the acetabular cup is located at the predetermined cup placing location, a current pose of the acetabular cup is locked; in response to a determination that the acetabular cup is not located at the predetermined cup placing location, the above operations until the acetabular cup, after pose adjustment, is located at the predetermined cup placing location.

In some embodiments, the pose adjustment signal is generated in response to detecting that the acetabular cup has completed the movement of a predetermined step length. In some embodiments, the robotic arm is controlled to drive the acetabular cup whose pose has been adjusted toward the predetermined cup placing location, and the pose adjustment signal is generated in response to detecting that the robotic arm has driven the acetabular cup to complete the movement by a predetermined step length, and according to the pose adjustment signal, the current pose of the acetabular cup is adjusted so as to make the center axis of the acetabular cup after the pose adjustment coincide with the center axis of the acetabular socket. Whether the acetabular cup after the pose adjustment is located at the predetermined cup placing location is detected; in response to a determination that the acetabular cup is located at the predetermined cup placing location, the current pose of the acetabular cup may be locked; and in response to a determination that the acetabular cup is not located at the predetermined cup placing location, the acetabular cup after the pose adjustment may be driven to continue to move toward the predetermined cup placing location by a predetermined step length. The pose adjustment signal is generated when the movement by predetermined step length is completed, and the pose adjustment is performed on the acetabular cup in accordance with the pose adjustment signal, so as to make the center axis of the acetabular cup after the pose adjustment coincide with the center axis of the acetabular socket. Whether the acetabular cup after the pose adjustment is located at the predetermined cup placing location is detected; in response to a determination that the acetabular cup is located at the predetermined cup placing location, the current pose of the acetabular cup may be locked; in response to a determination that the acetabular cup is not located at the predetermined cup placing location, the above steps may be repeated until the acetabular cup after the pose adjustment is located at the predetermined cup placing location.

In some embodiments, coordinates of the predetermined cup placing location are set to be origin coordinates, and a direction from the predetermined cup placing location to an opening of the acetabular socket is positive. In response to a determination that longitudinal coordinates of the acetabular cup after the pose adjustment are less than longitudinal coordinates of the predetermined cup placing location, the robotic arm is controlled to drive the acetabular cup to move in the direction of the opening of the acetabular socket; in response to a determination that the longitudinal coordinates of the acetabular cup after the pose adjustment are greater than the longitudinal coordinates of the predetermined cup placing location, the robotic arm is controlled to drive the acetabular cup to move in a direction of the bottom of the acetabular socket.

In some embodiments, movement of the acetabular cup to the predetermined cup placing location may be controlled by single one automatic adjustment mode, and the center axis of the acetabular cup at the predetermined cup placing location coincides with the center axis of the acetabular socket. In other embodiments, the movement of the acetabular cup to the predetermined cup placing location may also be controlled by a mixture of two automatic adjustment modes, and the center axis of the acetabular cup at the predetermined cup placing location coincides with the center axis of the acetabular socket. In some embodiments, the acetabular cup may be moved to a vicinity of the predetermined cup placing location first by an automatic adjustment mode in which the pose adjustment signal is triggered by the external force, and then by an automatic adjustment mode in which the pose adjustment signal is generated based on the predetermined step length, the acetabular cup is controlled to move from the vicinity of the predetermined cup placing location to the predetermined cup placing location, and a center axis of the acetabular cup coincides with the center axis of the acetabular socket. In some embodiments, when the automatic adjustment mode of the pose adjustment signal is triggered by the external force to move the acetabular cup to the vicinity of the predetermined cup placing location, the acetabular cup moves back and forth in the vicinity of the predetermined cup placing location, so in response to detecting that the acetabular cup begins to move back and forth at the predetermined cup placing location, the current automatic adjustment mode may be switched to an automatic adjustment mode that generates the pose adjustment signal based on the predetermined step length.

In some embodiments, in response to detecting that the acetabular cup, after the pose adjustment, is located at the predetermined cup placing location, a pose of the robotic arm may be locked by a pose locking instruction, henceforth locking the pose of the acetabular cup, so as to facilitate the execution of a cup striking operation after the pose of the acetabular cup is locked.

The basic concepts have been described above, and it is apparent to those skilled in the art that the foregoing detailed disclosure is intended as an example only and does not constitute a limitation of the present disclosure. Although not explicitly stated here, those skilled in the art may make various modifications, improvements and amendments to the present disclosure. These alterations, improvements, and modifications are intended to be suggested by this disclosure, and are within the spirit and scope of the exemplary embodiments of this disclosure.

Moreover, certain terminology has been used to describe embodiments of the present disclosure. As in "an embodiment", "one embodiment", and/or " some embodiments" means a feature, structure, or characteristic associated with at least one embodiment of the present disclosure. Accordingly, it should be emphasized and noted that two or more references to "an embodiment" or "one embodiment", or "an alternative embodiment" in different locations in the present disclosure do not necessarily refer to the same embodiment. In addition, some features, structures, or features in the present disclosure of one or more embodiments may be appropriately combined.

Furthermore, it will be appreciated by those skilled in the art that aspects of the present disclosure may be illustrated and described by a number of patentable varieties or circumstances, including any new and useful process, machine, product, or combination of substances, or any of their new and useful improvements. Accordingly, all aspects of the present disclosure may be performed entirely by hardware, may be performed entirely by softwares (including firmware, resident softwares, microcode, etc.), or may be performed by a combination of hardware and softwares. The above hardware or softwares can be referred to as "data block", "module", "engine", "unit", "component" or "system". In addition, aspects of the present disclosure may appear as a computer product located in one or more computer-readable media, the product including computer-readable program code.

Computer storage media may comprise a propagated data signal with a computer program encoded within it, e.g., on a baseband or as part of a carrier. The propagation signal may have a variety of manifestations, including an electromagnetic form, an optical form, and the like, or suitable combinations thereof. The computer storage medium may be any computer-readable medium other than a computer-readable storage medium, which may be used by connecting to an instruction-executing system, device, or apparatus for communicating, propagating, or transmitting for use. The program code located on the computer storage medium may be disseminated via any suitable medium, including radio, cable, fiber optic cable, RF, or the like, or a combination of any of the foregoing.

The computer program code required for the operation of the various portions of the present disclosure may be written in any one or more of a number of programming languages, including object-oriented programming languages such as Java, Scala, Smalltalk, Eiffel, JADE, Emerald, C++, C#, VB.NET, Python, etc., conventional procedural programming languages such as C, Visual Basic, Fortran 2003, Perl, COBOL 2002, PHP, ABAP, dynamic programming languages such as Python, Ruby and Groovy, or other programming languages. The program code can be run entirely on the user's computer, or as a stand-alone package on the user's computer, or partially on the user's computer and partially on a remote computer, or entirely on a remote computer or server. In the latter case, the remote computer may be connected to the user's computer through any form of network, such as a local area network (LAN) or a wide area network (WAN), or connected to an external computer (e.g., via the Internet), the remote computer may be connected to the user's computer through any form of network, such as a local area network (LAN) or wide area network (WAN), or connected to an external computer (e.g., via the Internet) or in a cloud computing environment, or used as a service such as Software as a Service (SaaS).

In addition, the order of processing elements and sequences, the use of numerical letters, or the use of other names described herein are not intended to qualify the order of the processes and methods of the present disclosure unless expressly stated in the claims. Although the above disclosure discusses through various examples what is currently considered to be a variety of useful embodiments of the disclosure, it is to be understood that such detail is solely for that purpose, and that the appended claims are not limited to the disclosed embodiments, but, on the contrary, are intended to cover modifications and equivalent arrangements that are within the spirit and scope of the disclosed embodiments. For example, although the implementation of various components described above may be embodied in a hardware device, it may also be implemented as a software only solution, e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various embodiments. However, this disclosure does not mean that the present disclosure object requires more features than the features mentioned in the claims. Rather, claimed subject matter may lie in less than all features of a single foregoing disclosed embodiment.

Numbers describing the number of components, attributes, and properties are used in some embodiments, and it is to be understood that such numbers used in the description of embodiments are modified in some examples by the modifiers "about", "approximately", or "substantially". Unless otherwise noted, the terms "about," "approximate," or "approximately" indicates that a $\pm 20\%$ variation in the stated number is allowed. Correspondingly, in some embodiments, the numerical parameters used in the present disclosure and claims are approximations, which approximations are subject to change depending on the desired characteristics of individual embodiments. In some embodiments, the numerical parameters should take into account the specified number of valid digits and employ general place-keeping. While the numerical domains and parameters used to confirm the breadth of their ranges in some embodiments of the present disclosure are approximations, in specific embodiments, such values are set to be as precise as possible within a feasible range.

For each patent, patent application, patent application disclosure, and other material cited in the present disclosure, such as articles, books, specifications, publications, documents, or the like, the entire contents of which are hereby incorporated herein by reference. Excluded are application history documents that are inconsistent with or create a conflict with the contents of the present disclosure, as well as documents that limit the broadest scope of the claims of the present disclosure that are currently or hereafter appended to the present disclosure. It should be noted that to the extent that the descriptions, definitions, and/or use of terms in the materials appurtenant to the present disclosure are inconsistent with or in conflict with the content set forth herein, the descriptions, definitions, and/or use of terms in the present disclosure shall prevail.

At last, it should be understood that the embodiments described in the present disclosure are merely illustrative of the principles of the embodiments of the present disclosure. Other modifications that may be employed may be within the scope of the present disclosure. Thus, by way of example, but not of limitation, alternative configurations of the embodiments of the present disclosure may be utilized in accordance with the teachings herein. Accordingly, embodiments of the present disclosure are not limited to that precisely as shown and described.

## Claims

1. A method for controlling a surgical robot, comprising:

   obtaining pose information of an end tool; and
   providing first feedback information to an operator based on the pose information.

2. The method of claim 1, wherein the providing first feedback information to an operator based on the pose information includes:
   applying a first feedback force to the operator based on the pose information.

3. The method of claim 2, wherein the first feedback force varies nonlinearly based on the pose information.

4. The method of claim 3, wherein the applying a first feedback force to the operator based on the pose information includes:

   obtaining a current position and a current stiffness of the end tool;
   determining the first feedback force based on the current position and the current stiffness; and
   applying the first feedback force to the operator based on a movement direction of the end tool.

5. The method of claim 4, wherein a corresponding relationship involves between the current stiffness and the current position.

6. The method of claim 5, wherein the current stiffness is determined based on a distance-stiffness function.

7. The method of claim 6, wherein the distance-stiffness function is continuous in more than half of a value interval of an independent variable of the distance-stiffness function.

8. The method of claim 7, wherein a derivative of the distance-stiffness function is not less than a first predetermined value.

9. The method of claim 8, wherein the distance-stiffness function includes a point at where a first order derivative of the distance-stiffness function is discontinuous.

10. The method of claim 8, wherein the distance-stiffness function includes two points at where a second order derivative of the distance-stiffness function is discontinuous.

11. The method of claim 8, wherein a first order derivative of the distance-stiffness function in at least more than half of a first value interval of the independent variable of the distance-stiffness function is not greater than a second predetermined value; the first order derivative of the distance-stiffness function in at least more than half of a second value interval of the independent variable of the distance-stiffness function is greater than the second predetermined value; and the first order derivative of the distance-stiffness function in at least more than half of a third value interval of the independent variable of the distance-stiffness function, is not greater than the second predetermined value.

12. The method of claim 11, wherein the first value interval of the independent variable of the distance-stiffness function, the second value interval of values of the independent variable of the distance-stiffness function, and the third value interval of the independent variable of the distance-stiffness function are sequentially continuous.

13. The method of claim 12, wherein the independent variable of the distance-stiffness function is a distance equivalent of the current position in a first predetermined region with respect to a predetermined path, and the first value interval of the distance equivalent ranges from 0 to 2/3; the second value interval of the distance equivalent ranges from 2/3 to 5/6; and the third value interval of the distance equivalent ranges from 5/6 to 1.

14. The method of claim 1, wherein the providing first feedback information to an operator based on position information includes:
applying a first feedback torque to the operator based on the position information.

15. The method of claim 14, wherein the first feedback torque varies nonlinearly based on the position information.

16. The method of claim 15, wherein the applying a first feedback torque to the operator based on the position information includes:

obtaining a current angle and a current angular stiffness of the end tool;
determining the first feedback torque based on the current angle and the current angular stiffness; and
applying the first feedback torque to the operator based on a movement direction of the end tool.

17. The method of claim 16, wherein a corresponding relationship involves between the current angular stiffness and the current angle.

18. The method of claim 17, wherein the current angular stiffness is determined based on an angle-stiffness function.

19. The method of claim 18, wherein the angle-stiffness function is continuous.

20. The method of claim 19, wherein a derivative of the angle-stiffness function is not less than a third predetermined value.

21. The method of claim 19, wherein the angle-stiffness function includes a point at where a first order derivative of the angle-stiffness function is discontinuous.

22. The method of claim 19, wherein the angle-stiffness function includes two points at where a second order derivative of the angle-stiffness function is discontinuous.

23. The method of claim 19, wherein a first order derivative of the angle-stiffness function in at least more than half of a first value interval of the independent variable of the angle-stiffness function is not greater than a fourth predetermined value;, the first order derivative of the angle-stiffness function in at least more than half of a second value interval of the independent variable of the angle-stiffness function is greater than the fourth predetermined value; and the first order derivative of the stiffness function in at least more than half of a third value interval of the independent variable of the angle-stiffness function is not greater than the fourth predetermined value.

24. The method of claim 23, wherein the first value interval of the independent variable of the angle-stiffness function, the second value interval of the independent variable of the angle-stiffness function, and the third value interval of the independent variable of the angle-stiffness function are sequentially continuous.

25. The method of claim 24, wherein the independent variable of the angle-stiffness function is an angular equivalent of the current angle in a first predetermined region with respect to a predetermined path, and the first value interval of the angular equivalent ranges from 0 to 2/3; the second value interval of the angular equivalent ranges from 2/3 to 5/6; and the third value interval of the angular equivalent ranges from 5/6 to 1.

26. The method of any one of claims 1 to 25, wherein the providing first feedback information to an operator based on position information includes:
providing the first feedback information to the operator based on the position information when the end tool is located in a first predetermined region.

27. The method of claim 26, wherein a top of the first predetermined region is a target position of the end tool on an object, and the target position follows movement of the object.

28. The method of claim 26, wherein the first predetermined region includes a tapered region.

29. The method of claim 28, wherein a cone angle of the tapered region ranges from 20 degrees to 40 degrees.

30. The method of claim 28, wherein a height of the tapered region ranges from 100mm to 300mm.

31. The method of claim 26, further including:
in the first predetermined region, causing the end tool to provide second feedback information to the operator when the end tool moves.

32. The method of claim 31, wherein the second feedback information includes a second feedback force; and the causing the end tool to provide second feedback information to the operator when the end tool moves includes:

obtaining a current speed and a current damping of the end tool; and
determining the second feedback force based on the current speed and the current damping.

33. The method of claim 32, wherein a permissible speed of the end tool does not exceed a predetermined speed.

34. The method of claim 32, wherein a corresponding relationship involves between the current speed and the current damping.

35. The method of claim 34, wherein the current damping is determined based on a damping function.

36. The method of claim 34, wherein the damping function is continuous in more than half of a value interval of an independent variable of the damping function.

37. The method of claim 36, wherein a derivative of the damping function is not less than a fifth predetermined value.

38. The method of claim 36, wherein the damping function includes a point at where a first order derivative of the damping function is discontinuous.

39. The method of claim 36, wherein the damping function includes two points at where a second order derivative of the damping function is discontinuous.

40. The method of claim 36, wherein a first order derivative of the damping function in at least more than half of a first value interval of the independent variable of the damping function is not greater than a sixth predetermined value; the first order derivative of the damping function in at least more than half of a second value interval of the independent variable of the damping function is greater than the sixth predetermined value; and the first order derivative of the damping function in at least more than half of a third value interval of the independent variable of the damping function is not greater than the sixth predetermined value.

41. The method of claim 40, wherein the first value interval of the independent variable of the damping function, the second value interval of the independent variable of the damping function, and the third value interval of the independent variable of the damping function are sequentially continuous.

42. The method of claim 31, wherein the second feedback information includes a second feedback torque; and the causing the end tool to provide second feedback information to the operator when the end tool moves includes:

obtaining a current angular speed of the end tool and a current rotational damping corresponding to the current angular speed; and
determining the second feedback torque based on the current angular speed and the current rotational damping.

**43.** The method of claim 31, wherein the second feedback information is determined based on a biometric feature of an object.

**44.** The method of claim 26, further including:
outputting a warning message when the end tool fails to satisfy a predetermined condition.

**45.** The method of claim 44, wherein the outputting a warning message when the end tool fails to satisfy a predetermined condition includes:
outputting the warning message when the end tool fails to disengage from the first predetermined region in accordance with the predetermined path.

**46.** The method of claim 44, wherein the outputting a warning message when the end tool fails to satisfy a predetermined condition includes:
outputting the warning message when a force or torque subjected by the end tool is greater than a predetermined threshold.

**47.** The method of claim 46, wherein the end tool includes a six-dimensional force sensor, and the six-dimensional force sensor is configured to detect the force and/or torque subjected by the end tool.

**48.** The method of claim 1, wherein in response to detecting movement of an object, the method further includes:

obtaining a first initial pose of the end tool and a pose offset of the object;
determining a target pose offset of the end tool based on the first initial pose and the pose offset; and
adjusting the end tool to a target pose based on the target pose offset to control the end tool to actively follow the object as the object moves.

**49.** The method of claim 48, further including:

obtaining a first pose corresponding relationship between the end tool and a reference coordinate system; and
the adjusting the end tool to a target position based on the target pose offset includes:
determining the target pose based on the first pose corresponding relationship and the target pose offset.

**50.** The method of claim 48, further including:

obtaining a second initial pose of the object and a current pose of the object; and
determining the pose offset of the object based on the second initial pose and the current pose.

**51.** The method of claim 50, wherein the determining a target pose offset of the end tool based on the first initial pose and the pose offset includes:

determining a second pose corresponding relationship between the object and the end tool based on the first initial pose and the second initial pose; and
determining the target pose offset based on the pose offset and the second pose corresponding relationship.

**52.** The method of claim 1, wherein the end tool includes an acetabular cup and an object includes an acetabular socket; the method further includes:

detecting whether a position of the acetabular cup satisfies a first predetermined condition;
in response to a determination that the acetabular cup satisfies the first predetermined condition, calibrating the acetabular cup.

**53.** The method of claim 52, wherein the calibrating the acetabular cup includes:

detecting a force direction on the acetabular cup when a force on the acetabular cup is greater than a threshold value; and
performing a first operation when the force direction on the acetabular cup is not parallel to an axis of the acetabular socket; and
performing a second operation when the force direction on the acetabular cup is parallel to the axis of the acetabular socket.

54. The method of claim 53, wherein the first operation includes:

adjusting an axial orientation of the acetabular cup so that an axis of the acetabular cup is parallel to the axis of the acetabular socket; and

when a distance between the axis of the acetabular cup and the axis of the acetabular socket is greater than the threshold value, controlling the acetabular cup to move to the axis of the acetabular socket until the axis of the acetabular cup coincides or substantially coincides with the axis of the acetabular socket.

55. The method of claim 54, further including:

performing the second operation if the acetabular cup is subjected to a blocking force while moving to the axis of the acetabular socket; and

if the acetabular cup is not subjected to the blocking force while moving to the axis of the acetabular socket, completing calibration when the axis of the acetabular cup coincides or substantially coincides with the axis of the acetabular socket.

56. The method of any one of claims 53 to 55, wherein the second operation includes:

controlling the acetabular cup to move away from the acetabular socket in a direction parrel to the axis of the acetabular socket; and

when a force subjected by the acetabular cup is not greater than the threshold value, controlling the acetabular cup to move to the axis of the acetabular socket until the axis of the acetabular cup coincides or substantially coincides with the axis of the acetabular socket.

57. The method of claim 53, wherein the second operation further includes:

when the force subjected by the acetabular cup is not greater than the threshold value, controlling the acetabular cup to move to the axis of the acetabular socket until the axis of the acetabular cup coincides or substantially coincides with the axis of the acetabular socket.

58. A surgical robot, comprising:

a robotic arm device configured to control an end tool to move;

a positioning device configured to obtain first pose information of the end tool and second pose information of an object; and

a processor, configured to:

obtain the first pose information of the end tool; and

provide first feedback information to an operator based on the first pose information.

59. The surgical robot of claim 58, wherein the robotic arm device includes a base and an arm assembly; and the positioning device includes a positioning information obtaining structure and one or more markers, the marker comprising a first marker provided on the base and a third marker provided on the object.

60. The surgical robot of claim 59, wherein the positioning device further includes a second marker for indicating a pose of the end tool.

61. The surgical robot of claim 59, wherein the marker includes at least one of an optical array, an electronic positioning device, an electromagnetic positioning device, a GPS positioning device, a gyroscope, or an accelerometer.

62. The surgical robot of claim 58, wherein the end tool includes an acetabular file or acetabular cup.

63. A method for guiding movement of an end tool, comprising:

detecting a current position of an end tool;

in response to determining that the current position of the end tool is not on a predetermined path in a constraint space, determining a sub-constraint space corresponding to the current position of the end tool and a distance-

stiffness function corresponding to the sub-constraint space, wherein a center axis of the constraint space is the predetermined path of the end tool, and the constraint space includes at least two sub-constraint spaces nested together; and

outputting a first feedback force toward the predetermined path to the end tool based on the current position and the distance-stiffness function corresponding to the current position.

**64.** A surgical robot, comprising:

a robotic arm device configured to drive an end tool to move in response to an external force and to output a feedback force to the end tool;

a positioning device configured to obtain a current position of the end tool in a constraint space, wherein a center axis of the constraint space is a predetermined path of the end tool, the constraint space includes at least two sub-constraint spaces nested together; and

a processor configured to:

detect the current position of the end tool via the positioning device;

in response to determining that the current position of the end tool is not on the predetermined path in the constraint space, determine a sub-constraint space corresponding to the current position of the end tool and a distance -stiffness function corresponding to the sub-constraint space; and

output a first feedback force toward the predetermined path to the end tool via the robotic arm device based on the current position and a distance-stiffness function corresponding to the current position.

**65.** A method for adjusting a pose of an end tool in real time, comprising:

obtaining a first pose corresponding relationship between an end tool and a reference coordinate system, and a second pose corresponding relationship between the end tool and an object;

obtaining a pose offset of the object in response to detecting a change of a pose of the object, wherein the pose offset of the object is a displacement of a current pose of the object relative to an initial pose of the object; and

controlling the end tool to move based on the first pose corresponding relationship and the pose offset of the object so that a pose corresponding relationship between the end tool and the object is restored to the second pose corresponding relationship.

**66.** A surgical robot, comprising:

a first pose obtaining device, provided on an object and maintaining a fixed corresponding relationship with the object, configured to obtain a pose of the object;

a second pose obtaining device, provided on a robotic arm carrying an end tool or on an end tool, configured to obtain a pose of the end tool; and

a controller, configured to obtain a first pose corresponding relationship between the end tool and a reference coordinate system and a second pose corresponding relationship between the end tool and the object; obtain a pose offset of the object when a change of a pose of the object is detected; control the end tool to move based on the first pose corresponding relationship and the pose offset of the object so that a pose corresponding relationship between the end tool and the object is restored to the second pose corresponding relationship.

**67.** A method for automatically adjusting a pose of an acetabular cup, comprising:

S 1, controlling an acetabular cup to move from a current position to a predetermined cup placing position and detecting a position adjustment signal in real time, and in response to detecting the position adjustment signal, obtaining current pose information of the acetabular cup and current pose information of an acetabular socket of an object, the predetermined cup placing position being located in the acetabular socket;

S2, adjusting a pose of the acetabular cup based on the current pose information of the acetabular cup and the current pose information of the acetabular socket, so as to cause a center axis of the acetabular cup to coincide with a center axis of the acetabular socket; and

S3, in response to determining that the acetabular cup whose position has been adjusted is not at the predetermined cup placing position, repeating the operations S 1 and S2.

**68.** A surgical robot, comprising:

a motion module, comprising a robotic arm configured to drive an acetabular cup to move;

a pose obtaining module, comprising an acetabular cup pose unit and an acetabular socket pose unit, configured to obtain current pose information of an acetabular cup via the acetabular cup pose unit in real-time and current pose of an acetabular socket of an object via the acetabular socket pose unit in real-time; and

a controller, configured to adjust a pose of the acetabular cup based on the current pose information of the acetabular cup and the current pose information of the acetabular socket so that a center axis of the acetabular cup coincides with a center axis of the acetabular socket.

100

Robotic arm
device 110

Processor 130

Positioning
device 120

**FIG. 1**

200

Pose information of an end
tool may be obtained

210

First feedback information
may be provided to an
operator based on the pose
information

220

In a first predetermined region,
causing the end tool to
provide second feedback
information to the operator
when the end tool moves

230

Outputting warning
information when the end tool
fails to satisfy a
predetermined condition

240

FIG. 2

300

15°

310

C

B

A

330

320

D

**FIG. 3**

400

Up

Down

410

420(300)

430(300)

D

**FIG. 4**

500

A current position and a current stiffness of an end tool may be obtained, and a first feedback force may be determined based on the current position and the current stiffness

510

The first feedback force may be applied to the operator based on a movement direction of the end tool

520

**FIG. 5**

Distance stiffness

| Inner layer region 310 | Middle layer region 310 | Outer layer region 310 | Distance equivalent |

**FIG. 6**

700

A current angle and a current angular stiffness of an end tool may be obtained, and a first feedback torque may be determined based on the current angle and the current angular stiffness

710

The first feedback torque may be applied to an operator based on a movement direction of the end tool

720

**FIG. 7**

Angular stiffness

900

FIG. ■

FIG. 9

Damping coefficient

FIG. 10

**1100**

A current angular speed of an end tool and a current rotational damping corresponding to the current angular speed may be obtained ⟋ 1110

A second feedback torque based on the current speed and current damping may be obtained ⟋ 1120

**FIG. 11**

**1200**

A first initial pose of an end tool and a pose offset of an object may be obtained ⟋ 1210

A target pose offset of the end tool may be determined based on the first initial pose and the pose offset ⟋ 1220

The end tool may be adjusted to the target pose based on the target pose offset to control the end tool to actively follow the movement of the object ⟋ 1230

**FIG. 12**

Obtaining a second initial pose of an object and a current pose of the object — 1211

Determining a pose offset of the object based on the second initial pose and the current pose — 1212

**FIG. 13**

A second pose corresponding relationship between an object and an end tool may be determined based on a first initial pose and a second initial pose — 1221

A target pose offset may be determined based on a pose offset and the second pose corresponding relationship — 1222

**FIG. 14**

1500

When a force subject by an acetabular cup is greater than a threshold value, a force direction of the force on the acetabular cup may be detected

1510

1520

whether the force direction on the acetabular cup is parallel to an axis of an acetabular socket may be determined

NO

Yes

1530

1550

A first operation is performed

1531

Adjusting an axial direction of the acetabular cup so that an axis of the acetabular cup is parallel to the axis of the acetabular socket

When a distance between the axis of the acetabular cup and the axis of the acetabular socket is greater than a threshold value, the acetabular cup is controlled to move to the axis of the acetabular socket until the axis of the acetabular cup coincides or substantially coincides with the axis of the acetabular socket

1532

A second operation is performed

1551

Controlling the acetabular cup to move away from the acetabular socket in a direction parallel to the axis of the acetabular socket

1540

Yes

Whether the acetabular cup is subjected to a blocking force when moving to the axis of the acetabular socket may be determined

No

Calibration is completed when the axis of the acetabular socket coincides or substantially coincides with the axis of the acetabular socket

Controlling the acetabular cup to move to the axis of the acetabular socket when the force subjected by the acetabular cup is not greater than the threshold value until the axis of the acetabular socket coincides or substantially coincides with the axis of the acetabular socket

1552

FIG. 15

Axis of
acetabular
cup

Axis of
acetabular
socket

Force

Acetabular
cup

Acetabular
socket

**FIG. 16**

Axis of
acetabular
cup

Axis of
acetabular
socket

Acetabular
cup

Acetabular
socket

**FIG. 17**

Axis of
acetabular
cup

Axis of
acetabular
socket

Acetabular
cup

Acetabular
socket

**FIG. 18**

Axis of
acetabular
socket ( axis of
acetabular cup)

Acetabular
cup

Acetabular
socket

**FIG. 19**

FIG. 20

**2100**

A current position of an end tool may be detected, and in response to a determination that the current position of the end tool is not on a predetermined path in a constraint space, one or more sub-constraint spaces corresponding to the current position of the end tool and a distance stiffness function corresponding to each of the one or more sub-constraint spaces may be determined

~2110

A first feedback force toward the predetermined path to the end tool may be output based on the current position and the distance-stiffness function corresponding to the current position

~2120

**FIG. 21**

**2200**

A first pose corresponding relationship between an end tool and a reference coordinate system and a second pose corresponding relationship between the end tool and an object may be obtained

~2210

A pose offset of the object may be obtained when a change in a pose of the object is detected

~2220

The end tool may be controlled to move according to the first pose corresponding relationship and the pose offset of the object, so that a pose corresponding relationship between the end tool and the object is restored to the second pose corresponding relationship

~2230

**FIG. 22**

**2300**

Controller 2030

First pose obtaining device 2310

Second pose obtaining device 2320

**FIG. 23**

**2400**

An acetabular cup may be controlled to move from a current position toward a predetermined cup placing location, and a pose adjustment signal may be detected in real time, and in response to detecting the pose adjustment signal, current pose information of the acetabular cup and current pose information of an acetabular socket of a target object may be obtained, wherein the predetermined cup placing location is located in the acetabular socket

2410

A pose of the acetabular cup may be adjusted according to the current pose information of the acetabular cup and the current pose information of the acetabular socket, so as to make a center axis of the acetabular cup coincide with a center axis of the acetabular socket

2420

In response to a determination that the acetabular cup is not at the predetermined cup placing location after the pose adjustment, operations 2410 and 2420 may be repeated

2430

**FIG. 24**

2500

Movement
module
2510

Controller 2030

Pose obtaining
module

**FIG. 25**

## INTERNATIONAL SEARCH REPORT

| International application No. |
| --- |
| **PCT/CN2022/104671** |

**A.   CLASSIFICATION OF SUBJECT MATTER**

A61B 34/30(2016.01)i

According to International Patent Classification (IPC) or to both national classification and IPC

**B.   FIELDS SEARCHED**

Minimum documentation searched (classification system followed by classification symbols)

A61B

Documentation searched other than minimum documentation to the extent that such documents are included in the fields searched

Electronic data base consulted during the international search (name of data base and, where practicable, search terms used)

CNKI, CNPAT, WPI, EPODOC: 手术, 机器人, 位姿, 位置, 反馈, 力, 力矩, 非线性, 连续, 刚度, 距离, 函数, 导, 角度, 区域, 运动, 定位, 速度, 阻尼, 旋转, 角速度, 警, 偏移, 末端, 标记, 臂, surgical, robotic, pose, position, feedback, force, moment, non-linear, continuous, stiffness, distance, function, guidance, angle, region, motion, speed, damping, rotation, angular, velocity, alarm, offset, tip, marker, arm

**C.   DOCUMENTS CONSIDERED TO BE RELEVANT**

| Category* | Citation of document, with indication, where appropriate, of the relevant passages | Relevant to claim No. |
| --- | --- | --- |
| PX | CN 113509270 A (WUHAN UNITED IMAGING ZHIRONG MEDICAL TECHNOLOGY CO., LTD.) 19 October 2021 (2021-10-19)<br>  claims 1-13, and description, paragraphs [0027]-[0154], and figures 1-11 | 1-6, 15-19, 26-35, 42-47, 58-62 |
| PX | CN 113520601 A (WUHAN UNITED IMAGING ZHIRONG MEDICAL TECHNOLOGY CO., LTD.) 22 October 2021 (2021-10-22)<br>  claims 1-10, and description, paragraphs [0024]-[0102], and figures 1-6 | 48-51 |
| X | CN 101160104 A (MAKO SURGICAL CORP.) 09 April 2008 (2008-04-09)<br>  description, page 9, last paragraph to page 82, paragraph 1, and figures 1-52B | 1-51, 58-62 |
| A | US 2005182320 A1 (STIFTER, J. et al.) 18 August 2005 (2005-08-18)<br>  entire document | 1-51, 58-62 |
| A | CN 112809686 A (HANGZHOU LIUYEDAO ROBOT CO., LTD.) 18 May 2021 (2021-05-18)<br>  entire document | 1-51, 58-62 |

☑ Further documents are listed in the continuation of Box C.          ☑ See patent family annex.

| \*   Special categories of cited documents: | "T"   later document published after the international filing date or priority date and not in conflict with the application but cited to understand the principle or theory underlying the invention |
| --- | --- |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
| --- | --- |
| **15 September 2022** | **26 September 2022** |

| Name and mailing address of the ISA/CN | Authorized officer |
| --- | --- |
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2022/104671** |

| C. | DOCUMENTS CONSIDERED TO BE RELEVANT | |
| --- | --- | --- |
| Category* | Citation of document, with indication, where appropriate, of the relevant passages | Relevant to claim No. |
| A | US 2021015634 A1 (THE REGENTS OF THE UNIVERSITY OF MICHIGAN) 21 January 2021 (2021-01-21)<br>entire document | 1-51, 58-62 |
| A | CN 113069207 A (HANGZHOU JIANJIA ROBOTS CO., LTD.) 06 July 2021 (2021-07-06)<br>entire document | 1-51, 58-62 |
| A | CN 111870348 A (WUHAN UNITED IMAGING ZHIRONG MEDICAL TECHNOLOGY CO., LTD.) 03 November 2020 (2020-11-03)<br>entire document | 1-51, 58-62 |

Form PCT/ISA/210 (second sheet) (January 2015)

## INTERNATIONAL SEARCH REPORT

International application No.

**PCT/CN2022/104671**

**Box No. II        Observations where certain claims were found unsearchable (Continuation of item 2 of first sheet)**

This international search report has not been established in respect of certain claims under Article 17(2)(a) for the following reasons:

1. ☑    Claims Nos.: **52-57**
   because they relate to subject matter not required to be searched by this Authority, namely:

   [1]   Claims 52-57 set forth a method for controlling a surgical robot, comprising calibrating an acetabular cup such that the axis of the acetabular cup coincides with or substantially coincides with the axis of an acetabular fossa. The method is used for hip replacement surgery, and belongs to a method for treating a human or animal body by surgery or therapy. Therefore, claims 52-57 belong to the subject matter defined in PCT Rule 39.1(iv) that does not warrant a search conducted by the international searching authority.

2. ☐    Claims Nos.:
   because they relate to parts of the international application that do not comply with the prescribed requirements to such an extent that no meaningful international search can be carried out, specifically:

3. ☐    Claims Nos.:
   because they are dependent claims and are not drafted in accordance with the second and third sentences of Rule 6.4(a).

**Box No. III        Observations where unity of invention is lacking (Continuation of item 3 of first sheet)**

This International Searching Authority found multiple inventions in this international application, as follows:

[1]   (1) Independent claims 1 and 58: a method for controlling a surgical robot, and a surgical robot, relating to feeding back information by means of a pose of an end tool.

[2]   (2) Independent claims 63 and 64: an end tool movement guide method and a surgical robot, relating to a constraint space, a preset path, and a distance stiffness function feedback force.

[3]   (3) Independent claims 65 and 66: a real-time correction method for an end tool pose, and a surgical robot, relating to controlling the movement of the surgical robot by means of a pose offset of an end tool.

[4]   (4) Independent claims 67 and 68: a method for automatically adjusting a pose of an acetabular cup and a surgical robot, relating to adjusting a pose of the acetabular cup.

[5]   Every two of independent claims (1, 58), (63, 64), (65, 66), and (67, 68) do not share a corresponding special technical feature. That is, every two of the described four inventions are not technically linked. The four inventions do not have a same inventive concept, and therefore do not comply with PCT Rule 13.1.

Form PCT/ISA/210 (continuation of first sheet) (January 2015)

## INTERNATIONAL SEARCH REPORT

| International application No. |
|---|
| **PCT/CN2022/104671** |

**Box No. III**     **Observations where unity of invention is lacking (Continuation of item 3 of first sheet)**

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **1-62**

**Remark on Protest**     ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
Information on patent family members

International application No.

**PCT/CN2022/104671**

| Patent document cited in search report | | | Publication date (day/month/year) | Patent family member(s) | | | Publication date (day/month/year) |
|---|---|---|---|---|---|---|---|
| CN | 113509270 | A | 19 October 2021 | None | | | |
| CN | 113520601 | A | 22 October 2021 | None | | | |
| CN | 101160104 | A | 09 April 2008 | WO | 2006091494 | A1 | 31 August 2006 |
| | | | | EP | 1871267 | A1 | 02 January 2008 |
| | | | | EP | 3470040 | A1 | 17 April 2019 |
| | | | | CA | 2826925 | A1 | 31 August 2006 |
| | | | | JP | 2008538184 | A | 16 October 2008 |
| | | | | CA | 2598627 | A1 | 31 August 2006 |
| US | 2005182320 | A1 | 18 August 2005 | WO | 03096920 | A1 | 27 November 2003 |
| | | | | DE | 50310543 | D1 | 06 November 2008 |
| | | | | EP | 1507488 | A1 | 23 February 2005 |
| | | | | JP | 2005525868 | A | 02 September 2005 |
| | | | | AU | 2003215562 | A1 | 02 December 2003 |
| | | | | AT | 409006 | T | 15 October 2008 |
| CN | 112809686 | A | 18 May 2021 | None | | | |
| US | 2021015634 | A1 | 21 January 2021 | WO | 2019191722 | A1 | 03 October 2019 |
| CN | 113069207 | A | 06 July 2021 | None | | | |
| CN | 111870348 | A | 03 November 2020 | None | | | |

Form PCT/ISA/210 (patent family annex) (January 2015)

**REFERENCES CITED IN THE DESCRIPTION**

**Patent documents cited in the description**

- WO 202110778767 A
- WO 202110777345 A

- WO 202110778776 A